# EUROPEAN PATENT SPECIFICATION

(11) **EP 1 297 841 B1**
(45) Date of publication and mention of the grant of the patent: **05.05.2010**
(21) Application number: 01934500.8
(22) Date of filing: 01.06.2001
(51) Int. Cl.: A61K 38/20, A61K 31/573, A61K 38/22, A61K 48/00, A61K 45/00, A61K 31/455, A61K 31/165, A61P 43/00, A61P 3/10, G01N 33/15, G01N 33/50, C12N 15/12, C12N 5/07

(54) **PANCREATIC LANGERHANS BETA CELL PROLIFERATION PROMOTER AND APOPTOSIS INHIBITOR, AND SCREENING OF CANDIDATE COMPOUNDS FOR THE DRUGS**
PROMOTOR DER PROLIFERATION UND INHIBITOR DER APOPTOSE PANKREATISCHER LANGERHANS BETA-ZELLEN UND SCREENING VON ANWÄRTER-VERBINDUNGEN FÜR MEDIKAMENTE
PROMOTEUR DE LA PROLIFERATION DES CELLULES DE LANGERHANS BETA PANCREATIQUES ET INHIBITEUR D'APOPTOSE, AINSI QUE CRIBLAGE DE COMPOSES CANDIDATS POUR CES MEDICAMENTS

(30) Priority: 02.06.2000 JP 2000170447; 28.02.2001 JP 2001054072
(43) Date of publication of application: 02.04.2003
(73) Proprietor: Takeda Pharmaceutical Company Limited, Osaka 540-8645 (JP)
(72) Inventor: Okamoto, Hiroshi, Sendai-shi, Miyagi 989-3201 (JP)
(74) Representative: Prins, Adrianus Willem
(86) International application number: PCT/JP2001/004660
(87) International publication number: WO 2001/093899

(56) References cited:
- EP-A- 0 453 319
- EP-A- 0 847 757
- EP-A- 0 914 829
- EP-A1- 0 461 560
- EP-A1- 0 847 757
- WO-A-97/16536
- WO-A-99/20740
- WO-A-99/46371
- JP-A- 6 219 963
- TAKAKO A; ET AL: "Activation of Reg gene, a gene for insulin-producing beta-cell regeration: poly(ADP-ribose)polymerase bins Reg promotor and regulates the transcription by autopoly(ADP-ribosyl)ation" PNAS, vol. 98, no. 1, 2 January 2001 (2001-01-02), pages 48-53, XP002380339
- GARCIA-OCANA A ET AL: "HEPATOCYTE GROWTH FACTOR OVEREXPRESSION IN THE ISLET OF TRANSGENIC MICE INCREASES BETA CELL PROLIFERATION, ENCHANCES ISLET MASS, AND INDUCES MILD HYPOGLYCEMIA" JOURNAL OF BIOLOGICAL CHEMISTRY, AMERICAN SOCIETY OF BIOLOCHEMICAL BIOLOGISTS, BIRMINGHAM,, US, vol. 275, no. 2, 14 January 2000 (2000-01-14), pages 1226-1232, XP002944870 ISSN: 0021-9258
- DUSETTI N J ET AL: "INDUCTION OF LITHOSTATHINE/REG MRNA EXPRESSION BY SERUM FROM RAT WITH ACUTE PANCREATITIS AND CYTOKINES IN PANCREATIC ACINAR AR-42J CELLS" ARCHIVES OF BIOCHEMISTRY AND BIOPHYSICS, NEW YORK, US, US, vol. 330, no. 1, 1 June 1996 (1996-06-01), pages 129-132, XP002944865 ISSN: 0003-9861
- OTONKOSKI T; MALLY MI; HAYEK A: "Oppoite Effects of beta-cell Differentiation and Growth on Reg Expression in Human Fetal Pancreatic Cells" DIABETES, vol. 43, 1994, pages 1164-1166, XP009066292
- BAEZA N; MORISCOT C; FIGARELLA C; GUY-CROTTE O; VIALETTES B: "REG PROTEIN: A POTENTIAL BETA_CELL_SPECIFIC GROWTH FACTOR?" DIABETES & METABOLISM, vol. 22, 1996, pages 229-234, XP009066317
- WATANABE T, ET AL: "Pancreatic beta-cell replication and amelioration of surgical diabetes by Reg protein" PROC. NATL. ACAD. SCI. USA, vol. 91, 1994, pages 3589-3592, XP002944867
- MIYAZAWA K, KITAMURA A, KITAMURA N: "Structural Organization and the Transcription initiation Site of the Human Hepatocyte Growt Factor Gene" BIOCHEMISTRY, vol. 30, 1991, pages 9170-9176, XP009066410
- DUSETTI NELSON J. ET AL.: 'Induction of lithostathine/reg mRNA expression by serum from rats with acute pancreatitis and cytokines in pancreatic acinar AR-42J cells' ARCH. BIOCHEM. BIOPHYS. vol. 330, no. 1, 1996, pages 129 - 132, XP002944865
- LIU YOUHUA ET AL.: 'Primary structure of rat HGF receptor and induced expression in glomerular mesangial cells' AM. J. PHYSIOL. vol. 271, no. 3, PART 2, 1996, pages F679 - F688, XP002944866
- WATANABE TAKUO ET AL.: 'Pancreatic beta-cell replication and amelioration of surgical diabetes by reg protein' PROC. NATL. ACAD. SCI. USA vol. 91, no. 9, 1994, pages 3589 - 3592, XP002944867
- GROSS D.J. ET AL.: 'Amelioration of diabetes in nonobese diabetic mice with advanced disease by linomide-induced immunoregulation combined with reg protein treatment' ENDOCRINOLOGY vol. 139, no. 5, 1998, pages 2369 - 2374, XP002944868
- CHAUHAN D. ET AL.: 'Harminder interleukin-6 inhibits fas-induced apoptosis and stress-activated protein kinase activation in multiple myeloma cells' BLOOD vol. 89, no. 1, 1997, pages 227 - 234, XP002944869
- GARCIA-OCANA A. ET AL.: 'Hepatocyte growth factor overexpression in the islet of transgenic increases beta cell proliferation, enhances islet mass and induces mild hypoglycemia' JOURNAL OF BIOLOGICAL CHEMISTRY vol. 275, no. 2, 2000, pages 1226 - 1232, XP002944870
- ZHANG LILIN J. ET AL.: 'Hepatocyte growth factor protects cultured rat cerebellar granule neurons from apoptosis via the phosphatidylinositol-3 kinase/akt pathway' NEUROSCI. RES. vol. 59, no. 4, 2000, pages 489 - 496, XP002944871
- DATABASE MEDLINE [Online] US NATIONAL LIBRARY OF MEDICINE, (BETHESDA, MD, USA) OKAMOTO H. ET AL.: 'The Reg gene family and Reg proteins: with special attention to the regeneration of pancreatic beta-cells', XP002907814 Retrieved from STN Database accession no. 99457321 & JOURNAL OF HEPATOBILIARY-PANCREATIC SURGERY vol. 6, no. 3, 1999, pages 254 - 262
- YUTAKA YONEMURA ET AL.: '(ADP-ribose) gousei kouso sogaizai ni yoru B saibou saisei' GENDAI IRYOU vol. 20, 1988, pages 297(1119) - 302(1124), XP002944872

## Description

### Technical Field

The present invention relates to the use of agents for proliferating pancreatic β-cells of Langerhans' islets, the use of agents for suppressing apoptosis of the cells, *in vitro* methods of proliferating pancreatic β-cells of the islets of Langerhans, and *in vitro* methods of suppressing apoptosis of said cells.

### Background Art

Pancreatic β-cells of the islets of Langerhans are the only cells that produce insulin in humans as well as in almost all animals, but they have a limited capacity for regeneration, which is a predisposing factor for the development of diabetes mellitus. Strategies for influencing the replication and growth of the β-cell mass are therefore important for the prevention and/or treatment of diabetes (Okamoto, H. (1985) Bioessays 2, 15-21).

The present inventors have established a model for islet regeneration in 90% depancreatized rats treated with poly (ADP-ribose) synthetase/polymerase (PARP) inhibitors such as nicotinamide and 3-aminobenzamide. In this model, regenerating islets in the remaining part of pancreas of PARP inhibitor-treated rats were markedly enlarged and consisted largely of insulin-producing β-cells, preventing the development of diabetes that would otherwise have been caused by the 90% pancreatectomy (Yonemura, Y. et al. (1984) Diabetes 33, 401-404).

In screening the regenerating islet-derived cDNA library, the inventors found a novel gene and named it Reg (regenerating gene) (Terazono, K. et al. (1988) J. Biol. Chem. 263, 2111-2114; Terazono, K et al. (1990) A novel gene, reg, expressed in regenerating islets, in "Molecular Biology of the Islets of Langerhans" (Okamoto, H., ed.), pp.301-313, Cambridge University Press, Cambridge; Yonekura, H. et al. (1994) Structure and expression of reg gene family in pancreatic islets, in "Frontiers of Insulin Secretion and Pancreatic B-Cell Research" (Flatt, R. and Lenzen, S. eds.), pp. 581-588, Smith-Gordon, London). The rat Reg cDNA encoded a 165-amino acid protein with a 21-amino acid signal peptide. The present inventors also isolated the human REG cDNA, which encoded a 166-amino acid protein with a 68% amino acid sequence identity to rat Reg protein. Recombinant rat Reg protein stimulated β-cell replication and increased the β-cell mass in the residual pancreas of 90% depancreatized rats, ameliorating the surgical diabetes (Watanabe, T. et al. (1994) Proc. Natl. Acad. Sci. USA 91, 3589-3592). Human REG protein also induced an expansion of the β-cell mass in non-obese diabetic (NOD) mice, resulting in the amelioration of diabetes (Gross, D. J. et al. (1998) Endocrinology 139, 2369-2374).

The present inventors have recently identified a Reg protein receptor (Kobayashi, S. et al. (2000) J. Biol. Chem. 275, 10723-10726) and found that the expression of the Reg receptor was not increased in regenerating islets as compared to that in normal islets, suggesting that the regeneration and proliferation of pancreatic β-cells for the increase of the β-cell mass are primarily regulated by the expression of Reg protein. This idea is consistent with the observations that Reg gene was first identified as a gene specifically expressed in regenerating islets (Terazono, K. et al. (1988) J. Biol. Chem. 263, 2111-2114; Terazono, K. et al. (1990) A novel gene, reg, expressed in regenerating islets. in "Molecular Biology of the Islets of Langerhans" (Okamoto, H., ed.), pp.301-313, Cambridge University Press, Cambridge; Yonekura, H. et al. (1994) Structure and expression of reggene family inpancreatic islets. in "Frontiers of Insulin Secretion and Pancreatic B-Cell Research" (Flatt, R. and Lenzen, S. eds.), pp. 581-588, Smith-Gordon, London) and that Reg gene expression was also observed in the phase of transient β-cell proliferation such as in pancreatic islets of BB/Wor/Tky rats during the remission phase of diabetes (Ishii, C. et al., (1993) Endocr. J. 40, 269-273), islets of NOD mice during active diabetogenesis (Baeza, N. J. et al. (1996) Diabetes 45, 67-70) and pancreatic ductal cells, which are thought to be progenitor cells of β-cells, during differentiation and Proliferation in a mouse model of autoimmune diabetes (Anastasi, E. et al.. (1999) Eur. J. Endocrinol. 141, 644-652).

As described above, there have been many reports on the involvement of the inhibition of PARP and expression of Reg gene in the regeneration and proliferation of β-cells. Therefore, the inhibition of PARP and induction of Reg gene expression are thought to be an important indicator for the development of therapeutic agents for disorders associated with the regeneration and proliferation of β-cells, such as diabetes.

However, mechanisms of regeneration and proliferation of β-cells including the correlation between the inhibition of PARP and Reg gene expression largely remain unsolved. Thus, the elucidation of such mechanisms has been demanded for the development of novel therapeutic agents for diabetes. On the other hand, it has been demonstrated that high concentrations of Reg protein induce the apoptosis of pancreatic β-cells (Kobayashi, S. et al. (2000) J. BioL Chem. 275,10723-10726). This suggests that methods for suppressing apoptosis of β-cells are expected to enable efficient proliferation the cells.

### Disclosure of the Invention

In view of these situations, the present invention has been made to provide novel use of agents for proliferating pancreatic β-cells of Langerhans' islets, use of agents for suppressing apoptosis of the cells, *in vitro* methods of proliferating pancreatic β-cells of Langerhans' islets, and in vitro methods of suppressing apoptosis of the cells. This invention also aims at elucidating the induction mechanism of Reg gene expression by the agents.

Furthermore , the present inventors found that hepatocyte growth factor (HGF) suppressed β- cell apoptosis induced by high concentrations of Reg protein and provides an agent for inducing expression of HGF gene. The use of HGF or HGF gene expression inducers enables to suppress apoptosis of β-cells, thereby enhancing the β-cell proliferation activity of Reg protein.

To resolve the above-mentioned problems, the present inventors have earnestly searched for agents that enhance Reg gene expression in β-cells, as a result, found that the use of interleukin (IL) -6, a cytokine whose receptor is gp130 together with glucocorticoid (dexamethasone) remarkably induces the expression of Reg gene in β-cells to promote their proliferation. Moreover, poly (ADP-ribose) synthetase/polymerase (PARP) inhibitors, such as nicotinamide and 3-aminobenzamide, further enhances this expression induction.

The inventors further investigated mechanisms of the induction of Reg gene expression by these agents and found that PARP, whose auto-ribosylation is inhibited by actions of these agents, binds to the GC box-like sequence in the 5' upstream region of Reg gene to induce the expression of this gene. Based on this finding, it has become possible to efficiently screen for candidate compounds for a Reg gene expression inducer using the activation of the upstream region of Reg gene induced by PARP binding to the GC box-like sequence as an indicator. This screening can be particularly preferably performed using a reporter system constructed by linking a reporter gene downstream of the GC box-like sequence-containing upstream region of Reg gene

The inventors also discovered that the addition of Reg protein together with HGF to β-cell line cells significantly increases the number of cells compared to the addition of Reg protein alone. Analysis of this HGF action revealed that HGF suppresses apoptosis induced by Reg protein. More surprisingly, it was revealed that the combined addition of the above-described cytokine, whose receptor is gp130, and glucocorticoid, which promotes Reg gene expression, also significantly enhances HGF gene expression. Similarly as in the case of Reg gene, the addition of PARP inhibitor (nicotinamide) further enhanced HGF gene expression. Thus, it has become evident that cytokine whose receptor is gp130 and glucocorticoid induce the expression of not only Reg gene but also HGF gene, thus extremely effectively increase β-cell mass while suppressing apoptosis induced by Reg protein.

The inventors further investigated mechanisms of the induction of HGF gene expression by these agents and succeeded in identifying the sequence essential for response to these agents in the promoter region of the gene. The use of this responsive sequence enables to efficiently screen for candidate compounds for HGF gene expression-inducers. This screening can be particularly preferably performed using a reporter system using a construct in which a reporter gene is linked downstream of the responsive sequence-containing upstream region of HGF gene.
Inflammatory mediators such as IL-6 and glucocorticoids and compounds isolated by screening for Reg or HGF gene expression inducers can be applied to the treatment and prophylaxis of diabetes.

Namely, the present invention relates to the use of agents for proliferating pancreatic β-cells of Langerhans' islets and agents for suppressing apoptosis of the cells, which agents comprises cytokine whose receptor is gp130 and glucocorticoid, *in vitro* methods of proliferating pancreatic β-cells of Langerhans' islets and *in vitro* methods of suppressing apoptosis of the cells, more specifically to:
(1) the use of a cytokine whose receptor is gp 130 in combination with a glucocorticoid in preparation of a medicament for proliferating pancreatic β-cells of Langerhans' islets;
(2) the use of a cytokine whose receptor is gp130 in combination with a glucocorticoid in preparation of a medicament for suppressing apoptosis of pancreatic β-cells of Langerhans' islets;
(3) the use of hepatocyte growth factor (HGF) or a nucleic acid coding for HGF in combination with Reg protein or a nucleic acid coding for Reg proteins in preparation of a medicament for proliferating pancreatic β-cells of Langerhans' islets;
(4) the use of hepatocyte growth factor (HGF) or a nucleic acid coding for HGF in combination with Reg protein or a nucleic acid coding for Reg protein in preparation of a medicament for suppressing apoptosis of pancreatic β-cells of Langerhans' islets;
(5) the use according to (1) or (2), wherein said cytokine is any one of IL-6, IL-11, LIF, CNTF, OSM or CT-1;
(6) the use according to (1) or (2), wherein said cytokine is IL-6;
(7) the use according to (1) or (2), wherein said glucocorticoid is dexamethasone;
(8) the use according to (1) or (2), further comprising use of a poly(ADP-ribose) synthetase/polymerase (PARP) inhibitor;
(9) the use according to (8), wherein said poly(ADP-ribose) synthetase/polymerase (PARP) inhibitor is nicotinamide or 3-aminobenzamide;
(10) the use according to any one of (1) to (9), wherein said medicament is used in the treatment or prophylaxis of diabetes;
(11) an in vitro method of proliferating pancreatic β-cells of Langerhans' islets, said method comprises administrating a cytokine whose receptor is gp130 in combination with a glucocorticoid;
(12) an in vitro method of suppressing apoptosis of pancreatic β-cells of Langerhans' islets, said method comprises administrating a cytokine whose receptor is gp130 in combination with a glucocorticoid;
(13) an in vitro method of proliferating pancreatic β-cells of Langerhans' islets, said method comprises administrating hepatocyte growth factor (HGF) or a nucleic acid coding for HGF in combination with Reg protein or a nucleic acid coding for Reg protein;
(14) an in vitro method of suppressing apoptosis of pancreatic β-cells of Langerhans' islets, said method comprises administrating hepatocyte growth factor (HGF) or a nucleic acid coding for HGF in combination with Reg protein or a nucleic acid coding for Reg protein;
(15) the method according to (11) or (12), wherein said cytokine is any one of IL-6, IL-11, LIF, CNTF, OSM or CT-1;
(16) the method according to (11) or (12), wherein said cytokine is IL-6;
(17) the method according to (11) or (12), wherein said glucocorticoid is dexamethasone;
(18) the method according to (11) or (12), further comprising administrating a poly(ADP-ribose) synthetase/polymerase (PARP) inhibitor; and
(19) the method according to (18), wherein said poly(ADP-ribose) synthetase/polymerase (PARP) inhibitor is nicotinamide or 3-aminobenzamide.

In this invention, "cytokines" refer to a group of soluble factors mediating the intercellular signal transduction. Cytokines function principally in immune and hematopoietic systems. Herein, "glucocorticoids" include steroid hormones secreted from the adrenal cortex, and synthetic substances having similar actions as those of the steroid hormones.

As a "cytokine whose receptor is gp130", IL-6 can be preferably used. Other cytokines used in this invention, whose receptor is gp130 like IL-6, include IL-11, leukemia inhibitory factor (LIF), ciliary neurotrophic factor (CNTF), oncostatinM (OSM), and cardiotrophin 1 (CT-1) (Taga, T. and Kishimoto, T. (1992) FASEBJ. 6, 3387-3396; Pennica, D. et al. (1995) Proc. Natl. Acad. Sci. USA 92, 1142-1146). "Glucocorticoid" used in the agents of this invention includes steroid hormones secreted from the adrenal cortex, such as cortisol, corticosterone, and cortisone, and synthetic substances having similar actions as these hormones such as dexamethasone and prednisolone.

To enhance the induction of Reg gene expression, the use of the present invention may further comprise the use of a poly(ADP-ribose) synthetase/polymerase (PARP) inhibitor. There is no particular limitation in the PARP inhibitors as long as they are capable of inhibiting the auto-ribosylation of PARP, including, for example, benzamide, nicotinamide, 3-aminobenzamide, 3-methoxybenzamide, 3-nitrobenzamide, picolinic acid amide, theophylline, and 3-isobutyl-1-methylxanthine.

The agents used in the present invention induce the expression of at least either one of Reg and HGF genes, but preferably both of them. Expression induction of both Reg and HGF genes enables a simultaneous manifestation of cell proliferation action of Reg protein and action of HGF to suppress apoptosis inducible by high concentrations of Reg protein. Such agents are extremely useful for the proliferation of cells like pancreatic β-cells.

Preferable "Reg gene" whose expression is induced by the agents used in the present invention is derived from mammals. Examples of mammals are rats, mice, humans, pigs, etc., but not limited to them. There are three subclasses of Reg gene, types I, II, and III. Regardless of types, these genes become the target of the agents of this invention, as long as their expressions are induced by binding of PARP to their 5' upstream region.

Preferable "HGF gene" whose expression is induced by the agents used in this invention is derived from mammals. Examples of mammals are rats, mice, humans, pigs, etc., but not limited to them. Furthermore, HGF gene, a target of the agents of the present invention, includes genes coding for HGF family proteins such as HGF-like protein (HLP) (ACCESSION U37055, Waltz, S. E. et al., J. Biol. Chem. 271 (15), 9024-9032 (1996)); X84043 (Thery, C. et al, Dev. Genet 17 (1), 9 0-101 (1995));Y08734(Aberger, F.et al, Mech. Dev 54(1), 23-37(1966)); U57455; and macrophage stimulating protein, etc.

The agents used in this invention are capable of promoting their regeneration and proliferation of β-cells. Since type III Reg gene is involved in the regeneration of nerves and intestines, the agents used in the present invention can be used to the regeneration of these tissues. Furthermore, the agents used in this invention, when applied to patients with disorders such as diabetes, are expected of their therapeutic and preventive effects. Therefore, the "expression inducer of Reg gene" of this invention includes both reagents used for the purpose of test researches and such, and medicines serving for treatment, prophylaxis, and such of disorders.

HGF is a hepatocyte growth factor. Furthermore, it is produced in a variety of mesenchymal cells, targeting many of epithelial, nervous, and endothelial cells, some of mesenchymal cells, etc. HGF has, in addition to its cell proliferating activity, the cell movement promoting activity, and epithelialization (of luminal structure or the like) inducing activity. In the developmental stage, HGF, as a mediator for interaction between epithelia and mesenchyme , is involved in the formation of many visceral organs such as liver, kidney, and lung, placenta, and skeletal system. Since HGF functions also in adults as an organ regeneration factor to promote the regeneration of liver, kidney, lung, and alimentary tracts, it is applicable to organopathy, and also functions as a neurotrophic factor, angiogenic factor, etc. Therefore, the agents used in this invention may be used as an agent for treating or preventing organopathy, or as a reagent and medicine for organ regeneration. The agents containing HGF and HGF gene expression inducer used in the present invention include both reagents used for the purpose of test researches and such, and medicines intended for the treatment, prophylaxis, and such of disorders.

The present invention also provides the use of agents for proliferating pancreatic β-cells of Langerhans' islets, comprising cytokines whose receptor is gpl30 and glucocorticoids. Cytokines whose receptor is gp130 and glucocorticoids induce the expression of Reg and HGF genes; thereby inducing the β-cell proliferation by Reg protein while suppressing the apoptosis induction by Reg protein. The present invention also provides the use of agents for suppressing apoptosis, said agents comprising cytokines whose receptor is gp130 and glucocorticoids. Cytokines whose receptor is gp130 and glucocorticoids suppress apoptosis by inducing the expression of HGF gene. The apoptosis-suppressing agents of this invention are particularly preferably used for suppressing apoptosis of pancreatic β-cells of Langerhans' islets. Furthermore, the apoptosis-suppressing agents are especially useful for suppressing apoptosis induced by Reg protein.

Furthermore, the present invention relates to methods for inducing the expression of HGF gene and/or Reg gene, the method comprising the step of contacting cytokines whose receptor is gp130 and glucocorticoids with cells. This invention also relates to a method for promoting the proliferation of pancreatic β-cells of Langerhans' islets, and a method for suppressing apoptosis of the cells, the method comprising the step of contacting cytokines whose receptor is gp130 and glucocorticoids with the cells. This invention further relates to the use of cytokines whose receptor is gp130 and glucocorticoids for inducing the expression of HGF gene and/or Reg gene, promoting the proliferation of pancreatic β-cells of Langerhans' islets and suppressing apoptosis of the cells.

The present invention also provides the use of agents for suppressing apoptosis of pancreatic β-cells of Langerhans' islets, comprising HGF. Although Reg protein not only promotes the proliferation of β-cells but also induces their apoptosis, the addition of HGF to the cells enables suppression of this apoptosis. Therefore, HGF is useful as an apoptosis-suppressing agent. It is possible to suppress the cellular apoptosis either by contacting HGF with cells or allowing to express and/or secret HGF using an HGF expression vector. HGF used as an apoptosis-suppressing agent in this invention is preferably from mammals. Examples of mammals are rats, mice, humans, pigs etc., but are not limited to these animals. Moreover, the apoptosis-suppressing agents used in this invention may comprise, in place of HGF, HGF family proteins such as HGF-like protein (HLP) (ACCESSION U3 705 5, Waltz, S. E. et al., J. Biol. Chem. 271(15), 902 4-9032 (1996); X84043, Thery, C. et al , Dev. Genet. 17(1), 90-101 (1995); Y08734, Aberger, F. et al., Mech Dev. 54 (1), 23-37 (1999; U57455), macrophage stimulating protein, etc. as long as they have the activity to suppress apoptosis induced by Reg protein.

The apoptosis-suppressing agents used the present invention are preferably used for suppressing apoptosis- of pancreatic β-cells of Langerhans' islets in particular, and especially useful for suppressing apoptosis induced by Reg protein.

The present invention also provide the use of agents for proliferating pancreatic β-cells of Langerhans' islets, comprising HGF, which suppresses apoptosis induced by Reg protein, thereby assisting β-cell proliferating activity of Reg protein. Therefore, HGF is useful for proliferating (assisting proliferation of) β-cells. It is possible to enhance the cell proliferation induced by Reg protein through the contact of HGF with the cell, or expression and/or secretion of HGF using an HGF expression vector.

There is no particular limitation in HGF used as a β-cell proliferating agent of the present invention, and any HGF may be used, but mammalian HGFs are preferred similarly as described above (for example, human HGF, X16323, Seki T. et al., Biochem. Biophys. Res. Commun. 1990, 172: 321-7; Nakamura, T. et al., Nature 342, 440-443 (1989); Nakamura, T., Prog. Growth Factor Res. 3,67-85 (1991)); rat HGF, D90102, Tashiro, K et al., Proc. Natl. Acad. Sci. U.S.A. 87, 3200-3204 (1990); Xenopus HGF, S77422, Nakamura, H. et al., Mech. Dev. 49 (1-2), 123-131 (1995), and so on). Examples of mammals are rats, mice, humans, pigs, etc., but are not limited to these animals. Furthermore, HGF family proteins such as HGF-like protein (HLP) (ACCESSION U37055, Waltz, S. E. et al., J. Biol. Chem. 271 (15), 9024-9032 (1996); X84043, Thery, C. et al., Dev. Genet. 17 (1), 90-101 (1995) ; Y08734, Aberger, F. et al, Mech Dev. 54 (1), 23-37 (1996) ; U57455), macrophage stimulating protein, etc. can be replaced with HGF in the β-cell proliferating agents of this invention as long as they are capable of promoting β-cell proliferation by Reg protein.

Alternatively, the agents used in this invention comprise a nucleic acid coding for HGF in place of HGF. That is, the present invention provides the use of agents for suppressing apoptosis of pancreatic β-cells of Langerhans' islets and agents for proliferating the cells, both comprising a nucleic acid coding for HGF. The nucleic acid may be either DNA or RNA HGF is expressed, for example, by inserting a nucleic acid coding for HGF into known vectors such as plasmid vectors or viral vectors, and introducing the resulting recombinant vectors into target cells. These vectors may be introduced into target cells whose apoptosis is intended to be suppressed, or other cells so that HGF can be allowed to be secreted from the recombinant cells and contact with target cells. Examples of vectors are pCDM8, pcDNA1.1, pZeoSV2 (all the above available from Invitrogen, Carlsbad, CA), pRevTRE, pTRE2, pShuttle, pTRE-Shuttle (all the above available from Clontech, Palo Alto , CA), pSI, pCI, pCI-neo (all the above available from Promega, Madison, WI), pCMV-Script, pBK-CMV, pBK-RSV {all the above available from Stratagene, La Jolla, CA) , but are not limited to them. Administration of vectors into living bodies may be performed either *in vivo* or *ex vivo.*

The above-mentioned agents comprising HGF or a nucleic acid coding for HGF may further contain Reg protein or a nucleic acid coding for Reg protein, thereby enabling the effective induction of β-cell proliferation independently from the endogenous Reg protein.

The present invention also provides a method of promoting the proliferation of pancreatic β-cells of Langerhans' islets, and a method of suppressing apoptosis of the β-cells comprising the step of contacting HGF with cells or introducing a nucleic acid coding for HGF into cells. β-Cells can be proliferated, for example, by contacting Reg protein with cells, or introducing a nucleic acid coding for Reg protein into cells. In addition to direct contact of Reg protein and/or HGF with cells, the contact of Reg protein and/or HGF with cells may be carried out, for example, by co-culturing the cells with other cells secreting Reg protein and/or HGF protein. Furthermore, the present invention provides the uses of HGF or nucleic acids coding for HGF to suppress apoptosis of pancreatic β-cells of Langerhans' islets, and promote proliferation of the β-cells. The agents containing HGF or a nucleic acid coding for HGF can be used as therapeutic or prophylactic agents for disorders such as diabetes.

Also disclosed is a method of screening for candidate compounds for expression inducer of Reg gene. This method is based on the findings of the present inventors that Reg gene expression is induced by binding of PARP to the 5' upstream region of Reg gene, and is performed by detecting the effect of test samples on PARP binding to the 5' upstream region of Reg gene using a reporter gene. This method enables efficient search for novel molecules that induce Reg gene expression other than the above-mentioned ones.

The first step of the screening method is to provide cells which have been transduced with a vector having a reporter gene operably linked to the PARP-binding sequence-containing 5' upstream region of Reg gene, (step (a)).

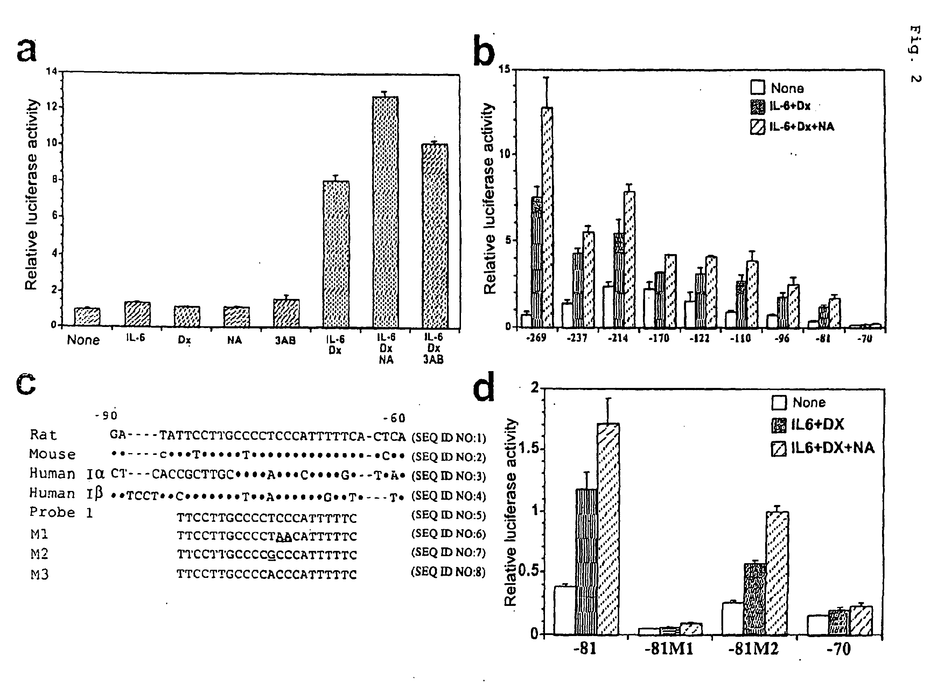

Southwestern analysis as described in Example 3 can identify "PARP binding sequence" in a reporter construct. Typically, this sequence contains the GC box-like sequence (Spl binding site) located in the 5' upstream region of Reg gene. As a PARP-binding sequence, for example, the -81 to -70 region (TGCCCCTCCCAT) of rat Reg gene is preferably used. Moreover, sequences corresponding to human REG Ia and REG Iβ (GCCCCCACCCCT and TGCTCCACCCAT, respectively) (cf. Fig. 2), their chimeric sequences, and such can also be used Furthermore, the "PARP-binding sequence- containing 5' -upstream region of Reg gene" in a reporter construct is not limited as long as the region contains the above-mentioned PARP binding sequence, and has the activity (promoter activity) to induce the transcription of the reporter gene linked downstream of the sequence. Any "reporter gene" can be used as long as their intracellular expressions can be detected. Examples of reporter genes are genes of luciferases (e.g. derived from firefly and sea pansy), β-galactosidase, chloramphenicol acetyl transferase, secretory alkaline phosphatase, etc. That the 5' upstream region of Reg gene and a reporter gene are "operably linked" means that, in a reporter construct, these two constitutive elements are so linked that the expression of the reporter gene is ensured to occur in response to the activation of the 5' upstream region of Reg gene.

Although there is no particular limitation in the vectors into which these constitutive elements are inserted, examples of preferred vectors are pSV0A/L-AΔ5' (Miyashita, H. et al. (1994) Biochim. Biophys. Acta 1219,241-243; Jeffrey, R.W.etal. (1987) Mol. Cell. Biol. 7, 725-737), pRL-null (Promega), pGL2-Basic (Proraega), pCAT3-Basic (Promega), pSEAP2-Basic (Clontech), pbetaGal-Basic {Clontech),etc.

Preferred cells to be transduced with the reporter construct are pancreatic β-cells (RINm5F, MIN6, βTC, INS-1, In111R1, HIT, and cells of isolated Langerhans' islets from various animals), and their progenitor cells (e.g. ARIP) . In the case of using the 5' upstream region of type III Reg gene in the reporter construct, cells derived from nervous system, intestinal epithelium, or liver may be used. Introduction of reporter constructs into cells can be performed by methods known to those skilled in the art such as the lipofection method, electroporation method, calcium phosphate method, and DEAE-dextran method.

In the next step of the screening method, cells provided in the step (a) are contacted with test samples to detect the reporter activity in the cells (step (b)).

Test samples include, for example, cell extracts, expression products of gene library, synthetic low molecular weight compounds, synthetic peptides , and natural compounds, though not limited to them. Reporter activity may be detected by methods known to those skilled in the art depending on the reporter gene. Cells can be contacted with not only test samples but also inflammatory mediators (the above-mentioned various cytokines whose receptor is gp130, glucocorticoids, PARP inhibitors, or any combinations thereof, etc.) to detect whether test samples further enhance effects of these inflammatory mediators or not.

Subsequently, in the screening method, compounds which enhance the reporter activity detected in the step (b) compared to the reporter activity detected in the absence of test samples are selected (step (c)).

When the reporter activity is significantly increased in cells contacted with test samples compared to the activity detected in the absence of test samples (control), a compound contained in test samples is judged to be a candidate compound for a Reg gene expression inducer.

Also disclosed is a method of screening for candidate compounds for an HGF gene expression inducer. This method is based on the finding of the present inventors that cytokines whose receptor is gp130 and glucocorticoids induce HGF gene expression and that a specific sequence in the promoter region of HGF gene is involved in this expression induction. The method is **characterized in that** the effect of test samples on the expression of a reporter gene is detected utilizing the reporter gene linked to the specific sequence designated "IL-6/dexamethasone (Dx) responsive sequence." This method enables the efficient search for novel molecules that induces HGF gene expression other than the above-mentioned ones.

In this screening method, the first step is to provide cells which have been transduced with a vector containing a reporter gene operably linked to the IL-6/Dx responsive sequence-containing 5' upstream region of HGF gene (step (a)).

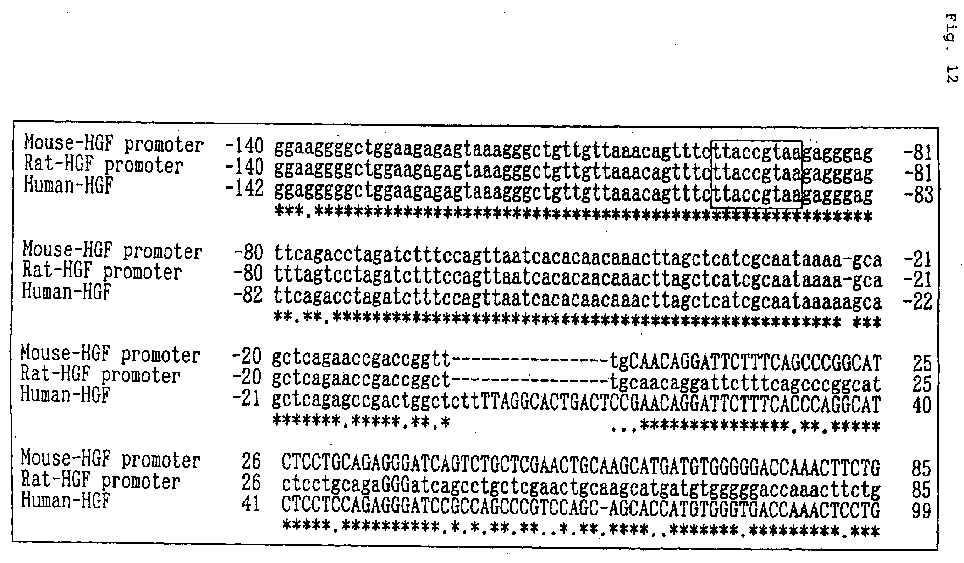

The "IL-6/Dx responsive sequence" refers to a sequence that is, in response to IL-6 and dexamethasone, responsible for inducing the expression of the gene located downstream of the sequence. A promoter analysis as described in Example 8 canidentify "IL-6/Dx responsive sequence" in a reporter construct. As an IL-6/Dx responsive sequence, for example, a fragment containing the -96 to -92 region (TTACC) of rat HGF gene, or corresponding fragments in the promoter region of HGF genes of other mammals including mice and humans, can be preferably used. Promoter sequences of rat HGF gene is known (Okajima, A., Miyazawa, K. and Kitamura, N. (1993) Fur. J. Biochem. 213, 113-119 (Ac. No. D14341); Aravamudan, B., Watabe, M. and Watabe, K. (1993) Biochem. Biophys. Res. Commun. 195, 346-353 (Ac. No. L23078)), mice (Liu, Y., Michalopoulos, G. K. and Zarnegar, R. (1994) J. Biol. Chem. 269 (6), 4152-4160 (Ac. No. L25131), and humans (Miyazawa, K., Kitamura, A and Kitamura, N. (1991) Biochemistry 30, 9170-9176 (Ac. No. M75967)), etc. (cf. Fig. 12). It is possible to perform the screening using these HGF promoter sequences.

A preferable example of an IL-6/Dx responsive sequence is, for example, a sequence containing the STATx consensus sequence (TTMCCRKAA; M: A or C, R: A or G, K: G or T) in the 5' upstream region of HGF gene. Positions of the STATx consensus sequence (TTACCGTAA) in the 5' upstream region of HGF gene of mice, rats, and humans are shown in Fig. 12. IL-6/Dx responsive sequences usually contain consecutive five nucleotides or more, for example, 8, 10, 12, 14, 16 or 18 nucleotides or more. There is no particular limitation in the "the IL-6/Dx responsive sequence-containing 5' upstream region of HGF gene" in the reporter construct as long as the region contains the IL-6/Dx responsive sequence, and has the activity (promoter activity) to induce the transcription of a reporter gene linked downstream of the sequence. There is no particular limitation in the "reporter genes" as long as their intracellular expressions are detectable. Similarly as described above, examples of reporter genes are genes of luciferases (e.g. derived from firefly and sea pansy), β-galactosidase, chloramphenicol acetyl transferase, secretory alkaline phosphatase, etc.. That the 5' upstream region of HGF gene and reporter gene are "operably linked" means that, in the reporter construct, these two constitutive elements are so linked that the expression of the reporter gene is ensured in response to the activation of 5' upstream region of HGF gene. Although there is no particular limitation in the vectors into which these constitutive elements will be inserted, examples of preferred vectors are the above-described pSV0A/L-AΔ5', pRL-null, pGL2-Basic, pCAT3-Basic, pSEAP2-Basic, pbetaGal-Basic, etc.

The cell to be transduced with a reporter construct are preferably pancreatic β-cells (RINm5F, MIN6, βTC, INS-1, In111R1, HIT and Langerhans' islet cells isolated from various animals) and their progenitor cells (e.g. ARIP). Introduction of the reporter construct into cells can be carried out by methods known to those skilled in the art such as the lipofect ion method, electroporat ion method, calcium phosphate method, and DEAE-dextran method.

In the screening method, next, cells provided in the step (a) are contacted with test samples to detect the reporter activity in the cells (step (b)). Examples of test samples are the same as described above. Reporter activity may be detected by methods known to those skilled in the art depending on the reporter genes. When inflammatory mediators (above-mentioned various cytokines whose receptor is gp130, glucocorticoids, PARP inhibitors, or combinations thereof) are contacted with cells in addition to test samples, it is possible to detect whether test samples enhance effects of the inflammatory mediators or not.

In the subsequent step of the screening method, compounds which increase the reporter activity detected in the step (b) compared to the activity detected in the absence of test samples (step (c)), are selected. When the reporter activity is significantly increased in cells contacted with test samples compared to the activity detected in the absence of test samples (control), the compound contained in test samples is judged to be a candidate compound for an HGF gene expression inducer.

Compounds that induce Reg gene expression and isolated by the screening method can be used as a Reg gene expression inducer, an agent for proliferating cells such as β-cells, and a therapeutic and prophylactic agent for disorders such as diabetes.

In addition, compounds that induce HGF gene expression and isolated by the screening method can be used as an apoptosis-suppressing agent, an agent for proliferating cells such as β-cells, and, furthermore, a therapeutic and prophylactic agent for disorders such as diabetes. If compounds that induce the express ion of both Reg and HGF genes in particular are screened, it becomes possible to develop agents with the β-cell proliferation activity equivalent to or higher than the agents containing cytokines whose receptor is gp130 and glucocorticoids.

For medical use the Reg gene and/or HGF gene express ion inducers can be formulated into pharmaceutical preparations using known pharmaceutical methods and administered to patients. For example, effective ingredients of the expression induces of this invention (various cytokines whose receptor is gp130, glucocorticoids, PAR inhibitors) are appropriately combined with pharmacologically acceptable carriers or media, such as sterilized water, physiological saline, plant oil, emulsifier, suspending agent, surfactant, stabilizer, and such to formulate pharmaceutical preparations and administered to patients. The pharmaceutical preparations can be administered to patients by intra-arterial, intravenous, and subcutaneous injections, or intranasal, perbronchial, intramuscular or oral administration.

When the expression inducers are added to cultured cells, in general, various cytokines (e.g. IL-6) whose receptor is gp130 may be added to the culture medium or the like to a final concentration of approximately 50 U/ml to 5 x 105 U/ml, glucocorticoids (e.g. dexamethasone) approximately 1 nM to 10 µM, and PARP inhibitors (e.g. nicotinamide and 3-aminobenzamide) approximately 10 µM to 50 mM. Furthermore, in the case of administration of these expression inducers to patients, therapeutically effective doses can be calculated with reference to effective concentrations in the above-described cultured cells. However, the dosage must be appropriately determined considering various factors such as body weight, age, and symptoms of patients, administration method, and toxicity of active ingredients of the pharmaceutical agents. Expert physicians will appropriately select suitable doses.

For the medical use of the agents for suppressing apoptosis of β-cells and-agents for proliferating the cells according to the present invention containing HGF or a nucleic acid coding for HGF, they can be formulated into pharmaceutical preparations using known pharmaceutical preparation methods and administered to patients. For example, pharmaceutical preparations may be prepared by appropriately combining HGF with pharmacologically acceptable carriers or media, such as sterilized water, physiological saline, plant oil, emulsifier, suspending agent, surfactant, and stabilizer, and administered to patients. Alternatively, HGF can be expressed and/or secreted in cells by the gene transfer using an HGF expression vector, The pharmaceutical agents may be administered to patients, for example, by intra-arterial, intravenous, and subcutaneous injections, or intranasal, perbronchial, intramuscular, or oral administration.

When HGF is added to cultured cells, in general, it may be added to the culture medium or the like approximately to a final concentration of 0.1 to 1,000 ng/ml, preferably 1 to 100 ng/ml, more preferably 5 to 50 ng/ml as. For administration to patients, therapeutically effective doses can be calculated with reference to effective concentrations of HGF in the above-described cultured cells. The dosage can be appropriately determined considering various factors such as body weight, age, and symptoms of patients, administration method, and toxicity of active ingredients in the pharmaceutical agents.

### Brief Description of the Drawings

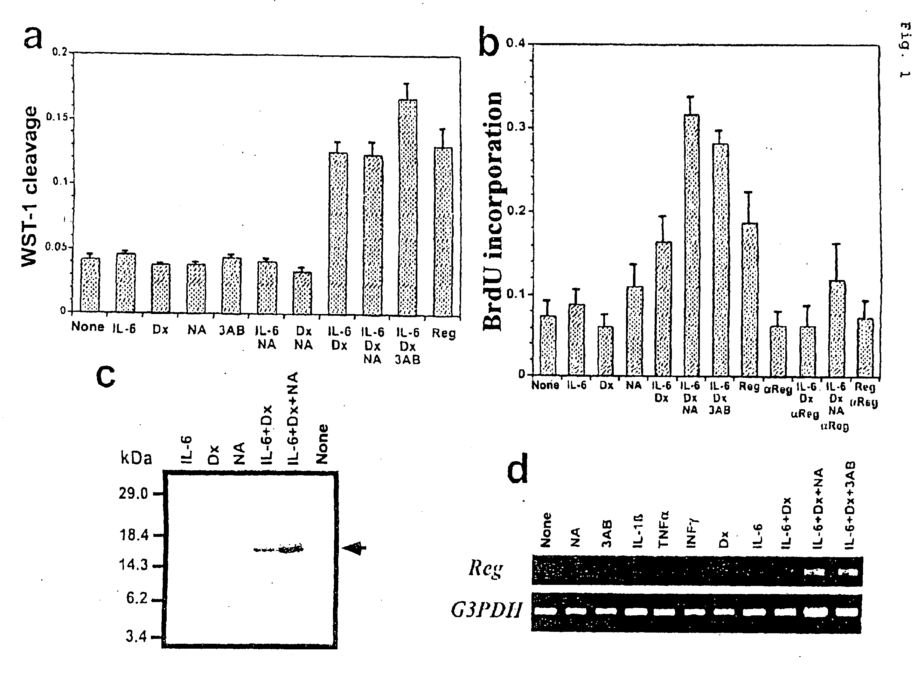

Fig. 1 shows graphs and photographs representing induction of Reg gene in RINm5F β-cells by IL-6/dexamethasone/PARP inhibitors. RINm5F cells were treated with IL-1β, TNFα, IL-6, dexamethasone (Dx), nicotinamide (NA) and/or 3-aminobenzamide (3AB).
   Panel a shows the results of WST-1 assay. The growth stimulation effect was analyzed by WST-1 cleavage.
   Panel b shows the results of BrdU incorporation. The stimulation of proliferation by Reg protein was analyzed by the incorporation of BrdU into RINm5F cells.
   Panel c is a photograph showing the results of immunoblot analysis. Secreted Reg protein was analyzed by immunoblot of the culture medium of the RINm5F cells.
   Panel d is a photograph showing the results of RT-PCR analysis. Expression of Reg gene and of the control glyceraldehyde-3-phosphate dehydrogenase (G3PDH) gene were analyzed by RT-PCR.
Fig. 2 shows identification of IL-6/dexamethasone/PARP inhibitor sensitive cis-element of Reg promoter.
   Panel a shows the results of induction of Reg/luciferase hybrid gene expression by IL-6, dexamethasone and PARP inhibitors.
   Panel b shows localization of IL-6/dexamethasone/nicotinamide response region in Reg promoter.
   Panel c shows alignment of promoter regions of type I Reg gene (Yonekura,H. et al. (1994) Structure and expression of reg gene family in pancreatic islets. in "Frontiers of Insulin Secretion and Pancreatic B-Cell. Research" (Flatt, R. and Lenzen, S. eds.), pp. 581-588, Smith-Gordon, London). Rat, mouse and human Reg gene promoter regions were aligned. Nucleotide substitutions in the IL-6/dexamethasone/nicotinamide response region are also indicated.
   Panel d shows identification of functional IL-6/dexamethasone/nicotinamide-responsive sequence within the Reg
   promoter by site-directed mutagenesis. Values represent mean±s e.m. of 3 to 8 independent transfection experiments. In each experiment, luciferase activities were expressed relative to the level of luciferase activity in untreated control cells, which was assigned a value of 1.0.

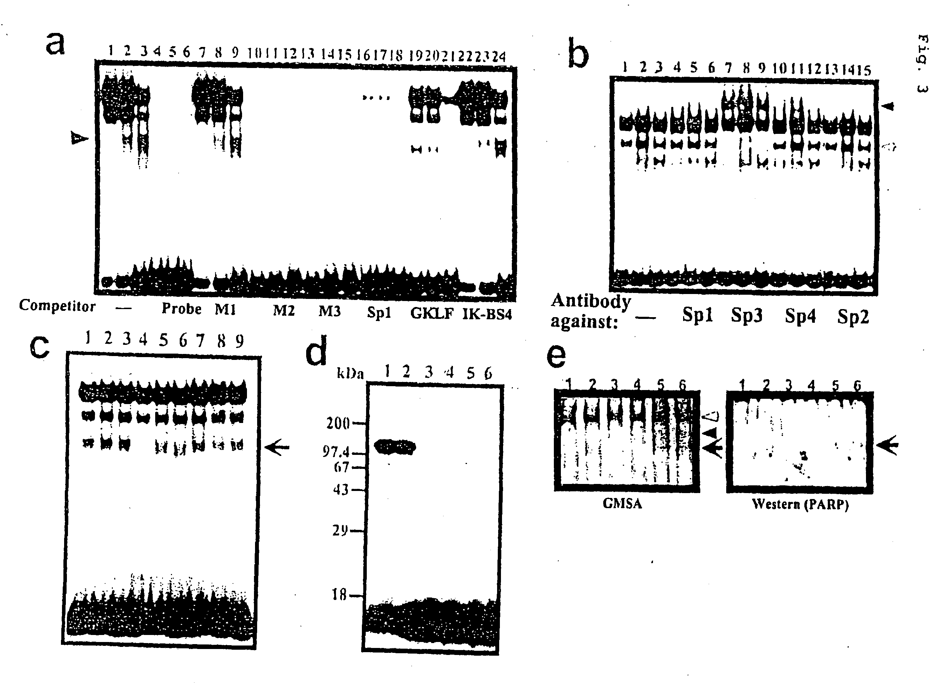

Fig. 3 shows photographs representing identification of nuclear protein specifically bound to IL-6/dexamethasone/nicotinamide-responsive element of Reg gene promoter.
   Panel a is a photograph showing binding of RINm5F nuclear extracts to the IL-6/dexamethasone/nicotinamide-responsive element by GMSA. Nuclear extracts from untreated cells were applied onto lanes 1, 4, 7, 10, 13, 16, 19 and 22, those from IL-6/dexamethasone treated cells were applied onto lanes 2,5,8,11,14,17,20 and 23 and those from IL-6/dexamethasone/nicotinamide treated cells were applied onto lanes 3, 6, 9, 12, 15, 18, 21 and 24.
   Panel b is a photograph showing supershift assay of IL-6/dexamethasone/nicotinamide-responsive element binding factors by antibodies against Sp1 (lanes 4 to 6), Sp2 (lanes 13 to 15), Sp3 (lanes 7 to 9) and Sp4 (lanes 10 to 12) . Nuclear extracts from untreated cells were applied onto lanes 1, 4, 7, 10 and 13, those from IL-6/dexamethasone treated cells were applied onto lanes 2, 5, 8, 11 and 14 and those from IL-6/dexamethasone/nicotinamide treated cells were applied onto lanes 3, 6, 9, 12 and 15.
   Panel c is a photograph showing effects of ADP-ribosylation of nuclear proteins on the binding ability to the IL-6/dexamethasone/nicotinamide-responsive element by GMSA. In the GMSA binding reaction, substrate, products and inhibitors of ADP-ribosylationwere added as follows: None (lane 1), 1 mM nicotinamide (lane 2.), 0.1 mM 3-aminobenzamide (lane 3), 1 mM β-NAD⁺ (lane 4), 1 mM β-NAD⁺ + 1 mM nicotinamide (lane 5), 1 mM β-NAD⁺ + 0.1 mM 3-aminobenzamide (lane 6), 1 mM α-NAD⁺ (lane 7), 1 mM ADP-ribose (lane 8) and 10 µM cyclic AUP-ribose (lane 9).
   Panel d is a photograph showing evidence of poly(ADP-ribosyl)ation in the GMSA binding reaction. [³²P]NAD' was added to the GMSA reaction and incubated with (lanes 2, 4 and 6) or without (lanes 1, 3 and 5) unlabeled Probe 1. Nicotinamide (1 mM, lanes 2 and 3) or 3-aminobenzamide (0.1 mM, lanes 4 and 5) was added in the incubation.
   Panel e is a photograph showing involvement of PARP in the poly(ADP-ribosyl)ation-sensitive DNA/protein complex. Nuclear extract of RINm5F control cells (lane 1). Nuclear extract of RINm5F cells treated with IL-6 (lane 2), dexamethasone (lane 3), nicotinamide (lane 4), IL-6/dexamethasone (lane 5) or IL-6/dexamethasone/nicotinamide (lane 6).

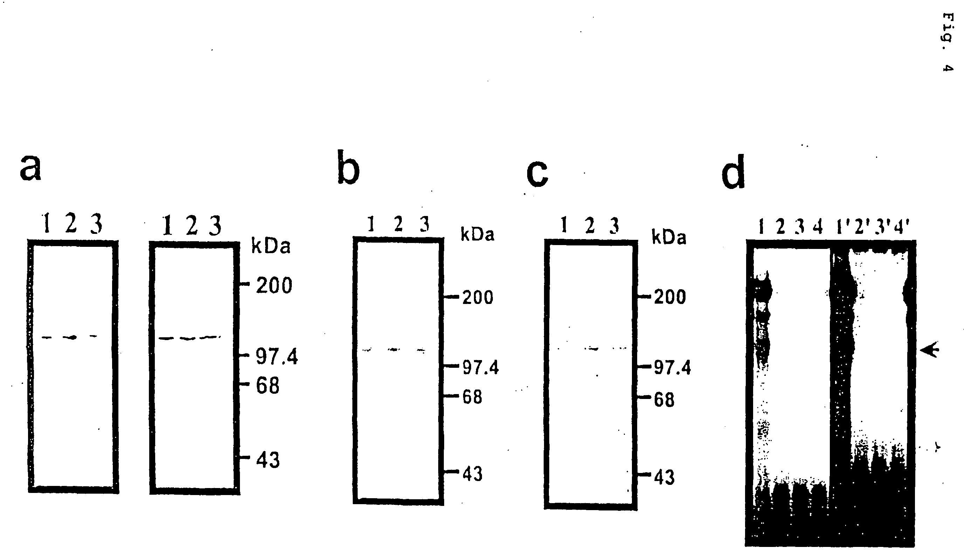

Fig. 4 shows identification of PARP as the IL-6/dexamethasone/nicotinamide-responsive element binding protein for Reg expression.
   Panel a is a photograph showing Southwestern and immunoblot analyses of RINm5F nuclear extracts. In the left panel, Southwestern blot analysis was performed using Probe 1. In the right panel, the blot was then probed by an anti-PARP antibody. Nuclear extracts from untreated, IL-6/dexamethasone and IL-6/dexamethasone/nicotinamide treated cells were applied to lanes 1, 2 and 3, respectively.
   Panel b is a photograph showing effect of poly (ADP-ribosyl) ation on the binding ability to the IL-6/dexamethasone/nicotinamide-responsive element. Nuclear extracts were separated by SDS-PAGE and transferred to a PVDFmembrane. The membrane was incubated with NAD⁺ (1 mM, lane 1), NAD⁺/nicotinamide (1 mM, lane 2) and NAD⁺/3-aminobenzamide (0.1 mM, lane 3), and probed by ³²P-labeled Probe 1.
   Panel c is a photograph showing binding of PARP to IL-6/dexamethasonc/nicotinamide-responsive element in Southwestern analysis. Purified PARP was separated by SDS-PAGE and transferred to PVDF membrane. The membrane was incubated with NAD⁺ (1 mM, lane 1), NAD⁺/nicotinamide (1 mM, lane 2) and NAD⁺/3-aminobenzamide (0.1 mM, lane 3), and probed by ³²P-labeled Probe 1.
   Panel d is a photograph showing binding of PARP to IL-6/dexamethasone/nicotinamide-responsive element in GMSA. Purified PARP was incubated in the presence of NAD⁺ (1 mM, lane 2), NAD⁺/nicoLinamide (1 mM, lane 3) and NAD⁺/3-aminobenzamide (0.1 mM, lane 4) with ³²P-Probe 1. Nuclear extract from IL-6/dexamethasone/nicotinamide treated cells was also analyzed as a control (lane 1). Right panel (lanes 1' to 4') represents the long exposure of the left panel (lanes 1 to 4).

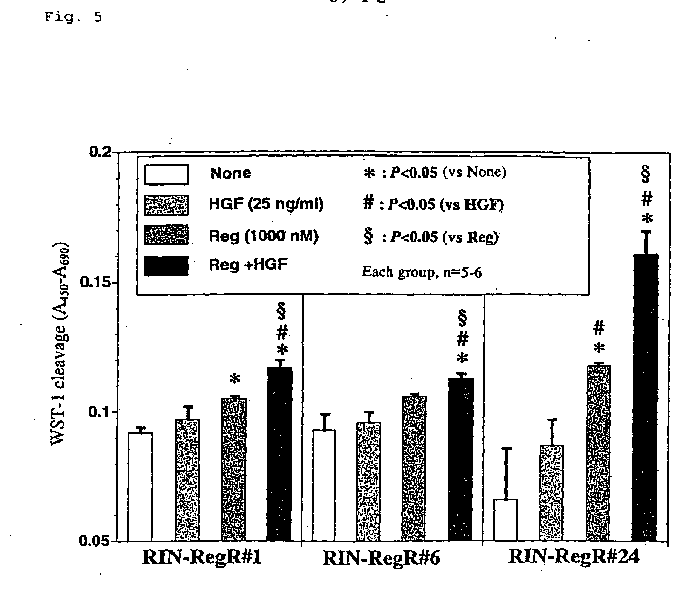

Fig. 5 shows the enhancement effect of HGF on the increase in the number of insulin-producing cells by Reg protein. Reg protein (1,000 nM), 25 ng/ml hepatocyte growth factor (HGF; Sigma), and both of them were added to RINm5F cell lines, #1, #6, and #24, which stably express the Reg protein receptor. After 24 h, cells were harvested to measure WST-1 cleavage. Graphs represent, from the left, results obtained by "None (no addition)," "addition of HGF (25 ng/ml) alone," "addition of Reg (1000 nM) alone," and "addition of Reg + HGF." HGF added alone did not increase the RINm5F cell number, but the combined addition of Reg protein and HGF significantly elevated the cell number. Significant differences by t test are also shown.

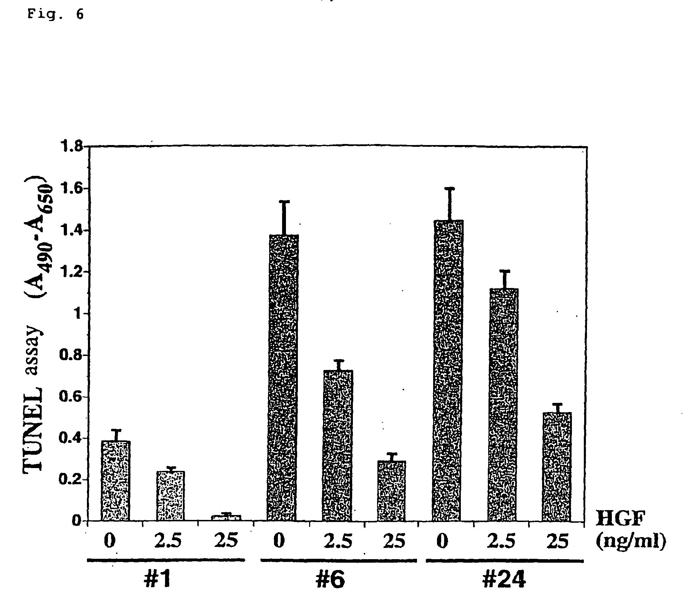

Fig. 6 shows the apoptosis-suppressive effect of HGF. Reg protein (1,000 nM) and varied concentrations (0, 2.5, and 25 ng/ml) of HGF were added to RINm5F cell lines, #1, #6, and #24, which stably express the Reg protein receptor, and, 24 h later, apoptosis was quantified by the TUNEL method. In every cell line, HGF concentration-dependently inhibited apoptosis induced by 1,000 nM Reg protein.

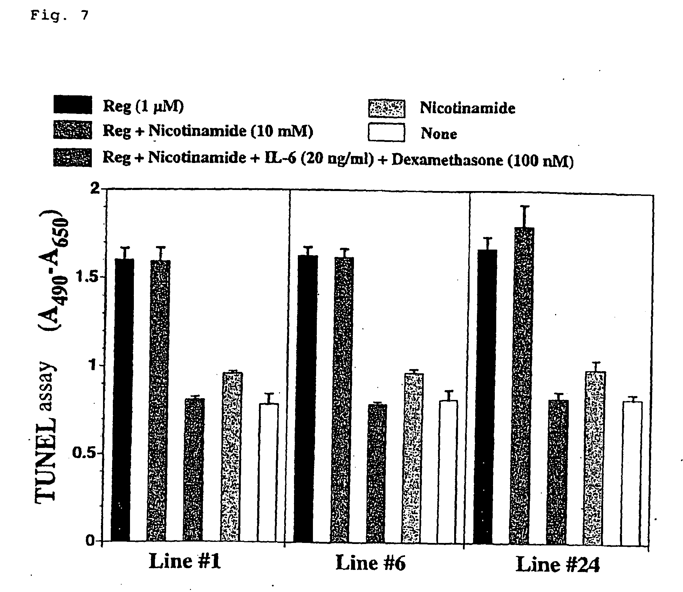

Fig. 7 shows apoptosis-suppressive effects of IL-6, glucocorticoid, and a poly(ADP-ribose) synthetase inhibitor. Reg protein (1,000 nM), IL-6 (20 ng/ml, 5,000 U/ml), dexamethasone (100 nM), and nicotinamide (10 mM), were added to RINm5F cell lines, #1, #6, and #24, which stably express the Reg protein receptor in the culture medium, and, 24 h later, apoptosis was quantified by the TUNEL method. Graphs show, from the left, the results obtained by "addition of Reg (1 µM)," "addition of Reg + nicotinamide (10 mM)," "addition of Reg + nicotinamide (10 mM) + IL-6 (20 ng/ml)," "addition of nicotinamide alone, "and "None (no addition) " The combined addition of dexamethasone and nicotinamide suppressed apoptosis induced by Reg protein, but nicotinamide alone did not.

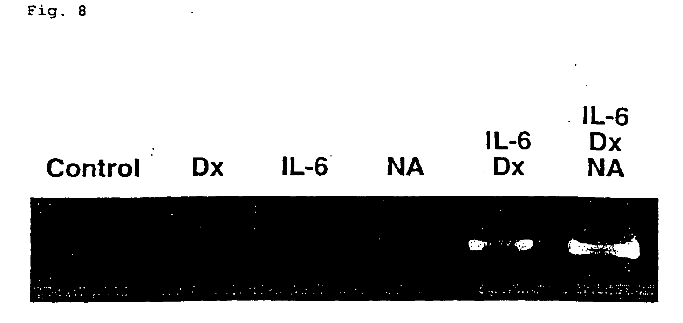

Fig. 8 is a photograph showing the expression of HGF induced by IL-6, glucocorticoid, and PARP inhibitor. The culture medium for RTNm5F cells was replaced with fresh medium containing 100 nM dexamethasone (Dx), 20 ng/ml IL-6 (5,000 U/ml) (IL-6), 10 mM nicotinamide (NA), or combinations thereof, and, after 24-h incubation, total RNA was prepared. RT-PCR was performed with this RNA as a template to detect HGF gene expression. The combined addition of IL-6 and dexamethasone induced the expression of HGF gene that was not usually expressed, and, moreover, the combined addition of nicotinamide, which is a PARP inhibitor, to IL-6/dexamethasone further enhanced the HGF gene expression.

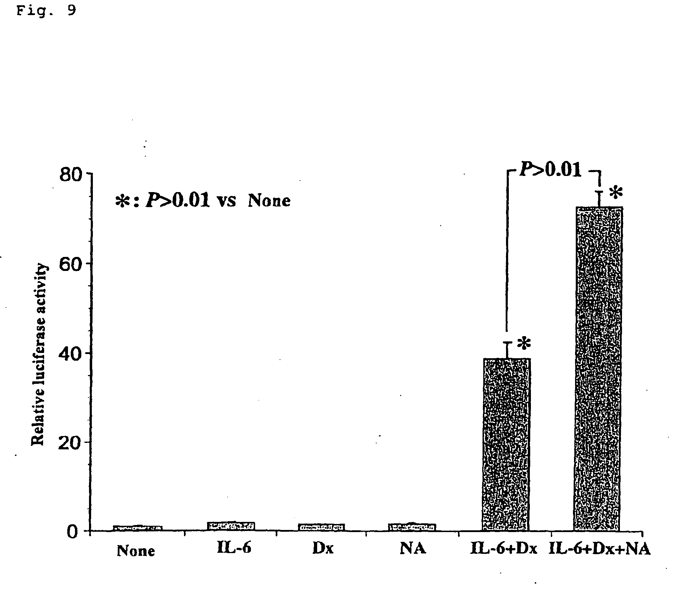

Fig. 9 shows the HGF transcriptional induction by IL-6, glucocorticoid, and PARP inhibitor. RINm5F cells were transduced with a construct in which the luciferase reporter gene had been linked downstream of the 5'-upstream region (-1336 to +85) of rat HGF gene, and, 24 h later, 20 ng/ml IL-6, 100 nM dexamethasone (Dx), and 10 mM nicotinamide (NA) were added to the culture medium, and further incubated for 24 h. Then, the cells were harvested to measure the luciferase activity. Values were normalized based on the protein concentration and gene transduction efficiency. The combined addition of IL-6 and dexamethasone induced the transcriptional activity of HGF gene. Moreover, the combined addition of nicotinamide, which is a poly(ADP-ribose) synthetase inhibitor, to IL-6/dexamethasone further enhanced this transcriptional induction.

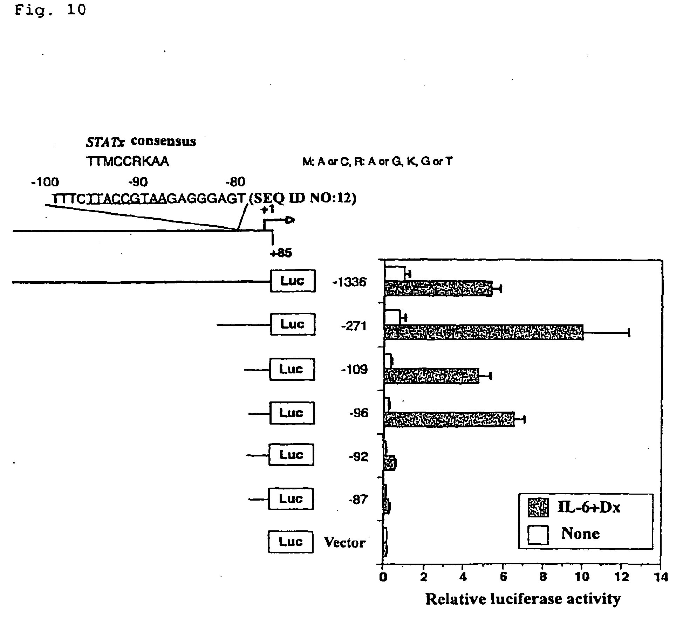

Fig. 10 shows identification of gene sequence essential for the expression of EiGFgene induced-by IL-6 and glucocorticoid using promoter assay. Luciferase reporter gene constructs were prepared using the 5' upstream region of rat HGF gene having various lengths. More specifically, DNA fragments corresponding to 1336 to +85, 271 to +85, 109 to +85, 96 to +85, 92 to +85, and 87 to +85 regions from the transcription initiation point of rat HGF gene (Okajima, A. et al. (1993) Eur. J. Biochem. 213, 113-119 (D14341)), were inserted into multi-cloning sites of pGL3-Basic coding for luciferase. These luciferase reporter constructs were introduced into RINm5F cells, and, 24 h later, 20 ng/ml IL-6 and 100 nM dexamethasone were added into the incubation medium, and further incubated for 24 h ("IL-6 + Dx").
   As a control, the cells were incubated without adding IL-6 and dexamethasone ("None")-. Then, the cells were harvested to measure the luciferase activity. As a result, plasmids containing the up to -96 nucleotide region upstream from the transcription initiation point of rat HGF gene induced the transcriptional activity by the combined addition of IL-6 and dexamethasone similarly as that containing the up to -1336 nucleotide upstream region, but plasmids containing the up to -92, and-87 nucleotide upstream region did not. The position of the binding sequence (TTMCCRKAA) for the STAT transcriptional factor family that had been detected in the -96 to -88 nucleotide upstream region from the transcription initiation point by TRANSFAC computer program (Heinemeyer, T. et al. (1998) Nucleic Acids Res. 26, 362-367) was underlined.

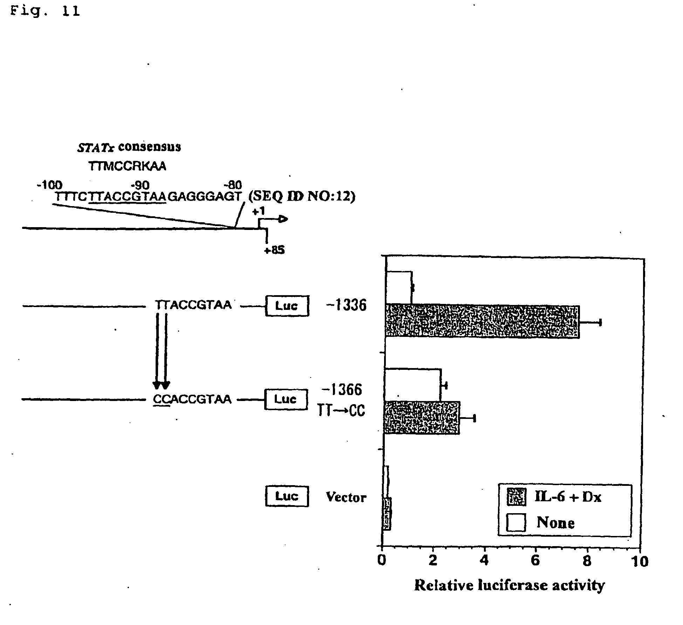

Fig. 11 shows identification of a gene sequence essential for the induction of HGF gene expression by IL-6 and glucocorticoid using promoter assay. A luciferase reporter gene construct was formed by replacing the two nucleotides TT in the STATx consensus sequence in the 5' upstream region (-1336 to +85) of rat HGF gene with CC. Similarly as in Fig. 10, luciferase reporter constructs were introduced into RINm5F cells, after 24 h incubation, 20 ng/ml IL-6 and 100 nM dexamethasone were added to the medium, and incubated for further 24 h ("IL-6 + Dx"). Then, the cells were harvested to measure luciferase activity. As a control, the cells were incubated without adding IL-6 and dexamethasone ("None"). The induction of transcriptional activity byIL-6 and dexamethasone was nullified in the transformed reporter gene construct. Therefore, the sequence TTACCGTAA, the-96 to -88 region of rat HGF, matching to the binding sequence for a STAT transcriptional regulatory factor family was thought essential for the transcriptional induction of HGF gene by IL-6 and dexamethasone.
Fig. 12 shows alignments of nucleotide sequences in 5' upstream regions of mouse ( 140 to +85; SEQ ID NO: 13), rat ( 140 to +85; SEQ ID NO: 14) and human ( 142 to +99 ; SEQ TD NO: 15) HGF genes. The STATx sequences are shown surrounded in square (corresponding to the 96 to 88 region in rat and mouse HGFs, and the 98 to 90 region in human HGF).

### Best mode for Carrying out the Invention

The present invention will be explained in detail below with reference to examples, but is not to be construed as being limited thereto. Any literatures cited herein are incorporated by reference.

### [Example 1] Induction of Reg gene expression

RINm5F cells, a rat insulinoma derived cell line, were maintained in RPMI 1640 medium supplemented with 10% fetal bovine serum (Bio Whittaker). For the stimulation experiments, RINm5F cells were seeded at 1.5 X 10⁶ cells/25 cm² flask and incubated for 24 h, then the medium was replaced with fresh medium containing the indicated amount of stimulants, 10 mM nicotinamide (Wako Pure Chemical Industries Ltd., Osaka, Japan), 2 mM 3-aminobenzamide (Chugai, Pharmaceutical Co., Ltd., Tokyo, Japan), 500 units/ml IL-1β (Sigma), 5,000 units/ml IL-6 (Genzyme, Cambridge, MA), 500 units/ml interferon (IFN) γ (Roche), 1,000 units/ml tumor necrosis factor (TNF) α (Roche), 100 nM dexamethasone (Sigma), or combinations thereof.

24 hours later, cells were harvested and WST-1 cleavage by mitochondrial dehydrogenases in viable cells and BrdU incorporation were measured as described in Kobayashi, S. et al. (2000) J. Biol. Chem., 275, 10723-10726 using a Cell Proliferation Reagent WST-1 (Roche) and a colorimetric cell proliferation ELISA kit (Roche), respectively.

As shown in Fig. 1a, the combined addition of IL-6/dexamethasone increased the number of RINm5F cells. Moreover, the combined addition of a PARP inhibitor such as nicotinamide or 3-aminobenzamide to IL-6/dexamethasone further increased the number of cells. In contrast, treatment with nicotinamide, 3-aminobenzamide, dexamethasone or IL-6 alone was ineffective. The treatment with IL-6/dexamethasone increased BrdU incorporation and the increase was attenuated by the addition of anti-Reg monoclonal antibody (Fig. 1b), suggesting that the growth stimulation by the addition of IL-6, dexamethasone and the PARP inhibitor is achieved by the increase of Reg protein in the medium.

The Reg protein levels in the RINm5F cultured media were examined after stimulation. After 24-hour incubation of the cells in a medium supplemented with stimulants, the medium was collected, and subjected to immunoblot analyses as described in Watanabe, T. et al. (1990) J. Biol. Chem., 265, 7432-7439. The medium of RINm5F cells was subjected to SDS polyacrylamide gel electrophoresis (SDS-PAGE) and electrotransferred to a PVDF membrane as described in Takasawa, S. et al. (1993) J. Biol. Chem., 268, 26052-26054. Western blotting was carried out using an anti-rat Reg monoclonal antibody (Terazono, K. et al. (1990) Diabetologia, 33, 250-252).

With no stimulants in the medium, RINm5F did not secrete Reg protein. IL-6 or dexamethasone alone also did not induce Reg protein secretion. On the other hand, the combination of IL-6/dexamethasone induced marked Reg protein secretion (Fig. 1c).

The Reg gene expression was analyzed by RT-PCR. The cells were harvested after 24h of culturing in a medium supplemented with stimulants, and total RNA was prepared using cesium trifluoroacetate as described in Takasawa, S. et al. (1993) J. Biol. Chem., 268, 26052-26054. RT-PCR was performed using primers corresponding to nucleotides 23 to 43 and 572 to 593 of rat Reg mRNA (Terazono, K. et al. (1988) J. Biol. Chem., 263, 2111-2114). Treatment with nicotinamide, 3-aminobenzamide, IL-1β, TNFα, dexamethasone or IL-6 alone had no effect on Reg expression, and the combined addition of IL-6/dexamethasone induced marked Reg mRNA accumulation. Moreover, in the presence of the PARP inhibitor, the combined addition of IL-6 and dexamethasone resulted in a 2-fold enhancement of the Reg mRNA level (Fig. 1d), whereas the combination of the PARP inhibitor with either IL-6 or dexamethasone alone was ineffective (data not shown).

These observations are well fitted to the previous findings that islet regeneration was observed in 90% depancreatized rats receiving the PARP inhibitor such as nicotinamide and 3-aminobenzamide (Yonemura, Y. et al. (1984) Diabetes, 33, 401-404; Terazono, K. et al. (1988) J. Biol. Chem., 263, 2111-2114; Terazono, K. et al. (1990) A novel gene, reg, expressed in regenerating islets, in "Molecular Biology of the Islets of Langerhans" (Okamoto, H., ed.), pp.301-313, Cambridge University Press, Cambridge; Okamoto, H. (1990) The molecular basis of experimental diabetes, in "Molecular Biology of the Islets of Langerhans" (Okamoto, H., ed.), pp.209-231, CambridgeUniversity Press, Cambridge; Okamoto, H. et al. (1985) Poly (ADP-ribose) synthetase inhibitors induce islet B-cell regeneration in partially depancreatized rats, in "ADP-Ribosylaltion of Proteins" (Althaus, F. R. et al. eds.), pp. 410-416, Springer-Verlag, Heidelberg; Terazono, K. et al. (1990) Diabetologia, 33, 250-252), and that Reg gene was expressed in the regenerating islet β-cells but not in normal pancreatic islets (Terazono, K. et al. (1988) J. Biol. Chem., 263, 2111-2114; Terazono, K. et al . (1990) A novel gene, reg, expressed in regenerating islets, in "Molecular Biology of the Islets of Langerhans" (Okamoto, H., ed.), pp. 301-313, Cambridge University Press, Cambridge; Yonekura, H. et al. (1994) Structure and expression of reg gene family in pancreatic islets, in "Frontiers of Insulin Secretion and Pancreatic B-Cell Research" (Flatt, R. and Lenzen, S. eds.), pp. 581-588, Smith-Gordon, London).

### [Example 2] Identification of regulatory element

The presence of a functional promoter element responsive to stimulation by IL-6/dexamethasone/PARP inhibitor in the 5'-flanking region of the rat Reg gene (Miyashita , H. et al. (1994) Biochim. Biophys. Acta, 1219, 241-243) was tested by transient expression assays. A 2,303-bp fragment containing the 5'-flanking region of Reg gene was fused to the luciferase gene and transfected into RINm5F β-cells.

Specifically, luciferase reporter gene constructs were generated as described in Miyashita, H. et al. (1994) Biochim. Biophys. Acta, 1219, 241-243. RINm5F cells were seeded at 1 X 10⁵ cells/well in 24-well dish. After 48 h, 3.0 µg of test plasmid and 0.2 µg of pCMV-SPORT β-gal (Life Technologies, Inc.) were transfected by lipofection (Felgner, J. H. et al. (1994) J. Biol. Chem., 269, 2550-2561) using DMRIE-C^{™} (Life Technologies, Inc.). After 24 h, the medium of each dish was replaced with fresh medium containing stimulants and incubated further for 24 h. Cells were harvested in 1 ml of ice-cold phosphate-buffered saline (PBS), washed twice with PBS, and extracts were prepared in extraction buffer (0.1 M potassium phosphate, pH 6.8, 1 mM DTT). The protein concentration was determined using a Coomassie reagent kit (Pierce) and bovine serum albumin as a standard. Luciferase activity was determined by a chemiluminescence substrate using a Pica Gene^{™} Luminescence Kit (Toyo Ink Mfg. Co, Ltd., Tokyo, Japan). β-galactosidase activity was determined using Aurora^{™} GAL-XE (ICN).

Fig. 2a compares luciferase activities determined in each treatment. Treatments with IL-6/dexamethasone, IL-6/dexamethasone/nicotinamide and IL-6/dexamethasone/3-aminobenzamide increased the luciferase activity 7, 14 and 12 times, respectively, whereas IL-6, dexamethasone, nicotinamide, 3-aminobenzamide alone, nicotinamide/IL-6 or nicotinamide/dexamethasone was ineffective.

In order to identify the region necessary for the induction of Reg gene by IL-6/dexamethasone/nicotinamide, progressive deletions of the Reg promoter were performed, and the resulting constructs were transfected into RINm5F cells. Deletions in the 5' to 3' direction resulted in a stepwise decrease of luciferase gene expression in the RINm5F cell line. The deletion down to position -269 did not alter significantly the expression of the reporter gene induced by the combined addition of IL-6 and dexamethasone. As shown in Fig. 2b, progressive deletion of the 188 nucleotides (to position-81) resulted in a gradual decrease of luciferase activity without altering the potent inducibility by IL-6/dexamethasone, but an additional deletion to nucleotide -70 caused a loss of the inducibility. These results suggest that the region between nucleotides -81 and -70 (TGCCCCTCCCA) is essential for the IL-6/dexamethasone-sensitive Reg promoter activity. The effect of nicotinamide to enhance the IL-6/dexamethasone-induced transcription of Reg gene was also abolished in the "-70" construct, whereas the enhancement was observed in the -2,303 to -81 deletions, suggesting that the enhancement of nicotinamide depends on the IL-6/dexamethasone-induced promoter activity.

A computer-assisted search for sequences similar to known cis-acting elements (Quandt, K. et al. (1995) Nucleic Acids Res., 23, 2878-2884; Heinemeyer, T. et al. (1998) Nucleic Acids Res., 26, 264-370) revealed that sequences similar to the GC box (Spl binding site) and the Ikaros binding element are present in the 11-bp region. Site-directed mutagenesis of the region from -81 to -70 was conducted within the luciferase construct of "-81" and the influence of the mutations on the amplitude of luciferase induction by IL-6/dexamethasone/nicotinamide was monitored. As shown in Fig. 2c and d, the site-directed mutation M1 (TGCCCCTCCCA -> TGCCCCTAACA), which altered the GC box, abolished the induction. The mutant M2 (TGCCCCTCCCA -> TGCCCCGCCCA) , which changed the Ikaros binding site to a classical GC box sequence, and the mutant M3 (TGCCCCTCCCA -> TGCCCCACCCA), which changed the sequence of the rat Reg (now termed Reg I (Miyashita, H. et al. (1994) Biochim. Biophys. Acta, 1219, 241-243)) promoter to those of human REG Iα (Watanabe, T. et al. (1990) J. Biol. Chem., 265, 7432-7439) and REG Iβ (Moriizumi, S. et al. (1994) Biochim. Biophys. Acta, 1217, 199-202), showed the induction by IL-6/dexamethasone, indicating that the GC box-like sequence (e.g. CCCCTCCC) is essential for conferring the IL-6/dexamethasone sensitivity and the enhancement by nicotinamide in Reg genes.

### [Example 3] PARP involvement in Reg transcription

Gel mobility shift assays (GMSA) using RINm5Fβ cells were carried out as follows. DNA probes for GMSA were synthesized as oligonucleotides. The sequences of the individual oligonucleotides in the sense orientation were as follows:
Probe 1, 5'-TTCCTTGCCCCTCCCATTTTTC-3' (SEQ ID NO: 5) corresponding to nucleotides -86 to -65 of rat Reg gene (Miyashita, H. et al. (1994) Biochim. Biophys. Acta, 1219, 241-243); Probe M1, 5'-TTCCTTGCCCCTAACATTTTTC-3' (SEQ ID NO: 6); Probe M2, 5'-TTCCTTGCCCCGCCCATTTTTC-3' (SEQ ID NO: 7); Sp1, 5'-ATTCGATCGGGGCGGGGCGAGC-3' (SEQ ID NO: 9) (Berg, J. M. (1992) Proc. Natl. Acad. Sci. USA, 89, 11109-11110); GKLF, 5'-AGGAGAAAGAAGGGCGTAGTATCTA-3' (SEQ ID NO: 10) (Zhang, W. et al. (1998) J. Biol. Chem., 273, 17917-17925); IK-BS4, 5'-TCAGCTTTTGGGAATGTATTCCCTGTCA-3' (SEQ ID NO: 11) (Molnar, A. and Georgopoulos, K. (1994) Mol. Cell. Biol., 14, 8292-8303).
Nuclear extracts from RINm5F cells were prepared as described (Miyashita, H. et al. (1994) Biochim. Biophys. Acta, 1219, 241-243) and stored at -80°C until use. GMSA was performed by the method of Garabedian (Garabedian, M. J. et al. Analysis of protein - DNA interactions, in Gene Transcription: A practical approach (Hamesm, B. D. , and Higgins, S. J. eds.), pp. 243-293, TRLPress, Oxford (1993)). The nuclear extract (10 µg) was incubated in a final volume of 20 µl of buffer containing 20 mM Tris-HCl (pH 7.9), 2 mM MgCl₂, 50 mM NaCl, 2 mM EDTA, 10% glycerol, 0.1% Nonidet P-40, 1 mM DTT, 50 µg/ml bovine serum albumin and 1 µg of poly (dI-dC). After incubation for 10 min at room temperature, 2.5 pmol of double-stranded DNA, previously labeled by an exchange reaction using MEGALABEL^{™} (Takara Shuzo) and [γ-³²P]ATP (Amersham Pharmacia Biotech), were added and incubated for 30 min at room temperature, then the binding reactions were resolved on pre-run 6% acrylamide gel. When competition experiments were conducted in the presence of 100-fold molar excess of cold probe, an unlabeled competitor was added with the labeled probe. In the supershift assay, 2 µg of anti-Sp1, -Sp2, -Sp3 or -Sp4 antibody (Santa Cruz) were added prior to the addition of the ³²P-probe. For the analysis of the effects of ADP-ribosylation, 0.2 µl of α-NAD⁺ (100 mM), β-NAD⁺ (100 mM), ADP-ribose (100 mM), cyclic ADP-ribose (1 mM), nicotinamide (100mM) and/or 3-aminobenzamide (10mM) were added prior to the addition of the ³²P-probe. A double-stranded DNA probe was also labeled using an ECL 3'-oligolabelling and detection system (Amersham Pharmacia Biotech), incubated with nuclear extract and electrophoresed as described above. After electrophoresis, DNA/proteins were electrophoretically transferred to a PolyScreen^{™} PVDF membrane (NEN) . DNA was detected by immunoblot using an ECL 3'-oligolabelling and detection system (Amersham Pharmacia Biotech).

As a result, multiple bands, representing the putative binding factors for the GC box-like sequence, were detected (Fig. 3a). The intensities of most bands appeared to be independent of the treatments of RINm5F cells. However, the lowest band was only detected in the nuclear extracts of RINm5F treated with IL-6/dexamethasone and with IL-6/dexamethasone/nicotinamide, and the intensity seemed to be correlated to the luciferase activity exhibited in the cells. All the bands disappeared by the addition of excéss amounts of unlabeled probe, the oligonucleotide competitor containing the M2 mutation (probe M2) and the GC box consensus oligonucleotide (Sp1) (Berg, J. M. (1992) Proc. Natl. Acad. Sci. USA, 89, 11109-11110). However, the competitor containing the M1 mutation (probe M1) could not competitively block the binding to the ³²P-labeled probe. The GKLF binding sequence (Zhang, W. et al. (1998) J. Biol. Chem., 273, 17917-17925) and Ikaros binding sequence (Molnar, A. and Georgopoulos, K. (1994) Mol. Cell. Biol., 14, 8292-8303) were also ineffective as competitors (Fig. 3a).

To investigate which Sp/XKLF transcription factor (Philipsen, S. and Suske, G. (1999) Nucleic Acids Res., 27, 2991-3000) binds to the sequence, a polyclonal antibody against Sp1, Sp2, Sp3 or Sp4 was used in a supershift assay in which RINm5F nuclear extracts were exposed to the antibody prior to incubation with the probe. As shown in Fig. 3b, the addition of Sp3 antibody led to the appearance of a supershift band, but the active transcriptional complex seen in the nuclear extracts of RINm5F cells stimulated with IL-6/dexamethasone and IL-6/dexamethasone/ nicotinamide was not supershifted, suggesting that a GC box/GC box-like sequence binding protein (s) other than Sp1, Sp2, Sp3 or Sp4 binds to the sequence to activate the Reg gene transcription.

The present inventors next analyzed the effects of ADP-ribosylation of nuclear proteins on the binding ability to the IL-6/dexamethasone/nicotinamide response element by GMSA. Poly (ADP-ribosyl) ation of nuclear proteins was performed in the same condition as GMSA in the presence of 62 nM [adenylate-³²P]NAD⁺ (2 x 10⁶ cpm, NEN). The ADP-ribosylated proteins were precipitated by the addition of 9 volume of acetone, separated by SDS-PAGE (12.5%) and autoradiographed.

As shown in Fig. 3c, the addition of β-NAD⁺, but not that of α-NAD' (Okamoto, H. (1970) Methods Enzymol., 18, 67-71), attenuated the IL-6/dexamethasone/nicotinamide-dependent band. Metabolites of β-NAD' such as ADP-ribose and cyclic ADP-ribose (Takasawa, S. et al. (1993) Science, 259, 370-373; Okamoto, H. et al. (2000) Physiological and pathophysiological significance of the CD38-cyclic ADP-ribose Signaling system, in "Human CD38 and Related Molecules" (Mehta, K. and Malavasi, F. eds.), pp. 121-145, Karger, Basel), did not attenuate the band intensity. Inhibitors of ADP-ribosylation reaction such as nicotinamide and 3-aminobenzamide quenched the effect of the addition of β-NAD⁺ and the concentrations of half maximal inhibition by nicotinamide and 3-aminobenzamide were estimated to be 250 µM and 30 µM, respectively. Since these concentrations of nicotinamide and 3-aminobenzamide inhibited poly(ADP-ribosyl)ation but not mono (ADP-ribosyl) ation (Rankin, P. W. et al. (1989) J. Biol. Chem., 264, 4312-4317), the inhibitory effect of β-NAD⁺ on the GMSA band through the poly(ADP-ribosyl)ation of nuclear proteins was indicated.

This was further supported by the poly (ADP-ribosyl) ation of PARP in the GMSA reaction (Fig. 3d) and by the involvement of PARP in the GMSA complex (Fig. 3e, lanes 5 and 6).

To determine the number and size of the proteins that directly interact with the GC box-like sequence in the -81 to -70 region of Reg gene, Southwestern experiments were carried out. Southwestern blot analysis was carried out as described in Jensen, D. E. et al. (1995) J. Biol. Chem., 270, 6555-6563. Nuclear extracts (25 µg) were subjected to SDS-PAGE (7%). The resolved proteins were transferred electrophoretically to Immobilon^{™} (Millipore). The blot was first washed twice (10 min each, room temperature) in 20 mM Hepes (pH 7.9), 3 mM MgCl₂, 40 mM KCl, 10 mM β-mercaptoethanol, blocked for 60 min in 20 mM Hepes (pH 7.9) containing 4% non-fat milk, and then washed once in binding buffer containing 10 mM Hepes (pH 7.9), 70 mM NaCl, 1 mM DTT, 0.3 mM MgCl₂, and 0.1% Triton X-100. Hybridization of the blot with radiolabeled Probe 1 (2 x 10⁵ cpm/ml) was performed for 3 h at room temperature in 40 µl/cm² binding buffer containing bovine serum albumin (60 µg/ml) and poly(dI-dC) (37.5 µg/ml). Prior to exposure of the blot to X-ray film, it was washed twice (5 min at room temperature) in binding buffer containing 0.01% Triton X-100. To evaluate the effect of poly(ADP-ribosyl)ation on DNA binding, the PARP activity was reconstituted on the membrane and the membrane was incubated with 1 mM NAD⁺ in the presence or absence of the PARP inhibitor (1 mM nicotinamide or 0 .1 mM 3-aminobenzamide) as described in Simonin, F. et al. (1991) Anal. Biochem., 195, 226-231, and then subjected to Southwestern analysis. As shown in Fig. 4a, a 113 kDa nuclear protein was found to bind the sequence, suggesting that PARP is the binding protein to the cis-regulatory element of Reg gene.

The membrane used for the Southwestern blot analysis was then incubated with two different antibodies against PARP (Clontech, C-2-10; and Upastate Biotechnology, 06-557). The 113 kDa band recognized by the antibodies was matched to the band identified by the Southwestern blot (Fig. 4a, right panel), suggesting the possible binding of PARP to the sequence in the Reg promoter.

Since no other poly(ADP-ribosyl)ated proteins than PARP itself were detected in the GMSA reaction (Fig. 3d), the effects of poly (ADP-ribosyl) ation on the ability of the cis-element binding were tested. Poly (ADP-ribosyl) ation of nuclear proteins was performed in the same condition as GMSA in the presence of 62 nM [adenylate-³²P] NAD⁺ (2 x 10⁶ cpm, NEN). The ADP-ribosylated proteins were precipitated by the addition of 9 volume of acetone, separated by SDS-PAGE (12.5%) and autoradiographed. The cis-element binding ability of the 113 kDa protein appeared to be independent of the poly (ADP-ribosyl) ation (Fig. 4b).

In addition, using purified human PARP (40 ng, Trevigen Inc.) instead of nuclear extracts, Southwestern blot analysis was performed as described above. The purified recombinant PARP also showed the binding to the cis-element which was also independent of the poly (ADP-ribosyl) ation of PARP (Fig. 4c), indicating that PARP acts as a cis-element binding protein.

GMSA was carried out using purified human PARP (10 ng, Trevigen Inc., Gaithersburg, MD) as described above. The purified PARP formed a retarded band in GMSA and the position of the band was different from that of the active transcriptional DNA/protein complex in RINm5F nuclear extract (Fig. 4d), suggesting that the DNA/protein complex contains other nuclear proteins than PARP and that poly(ADP-ribosyl)ation of PARP may attenuate the interaction with the other proteins.

### [Example 4] Enhancement effect of HGF on incrcase in number of insulin-producing cells by Reg protein

Rat insulinoma derived RINm5F cell lines, #1, #6, and #24, which stably express the Reg protein receptor (Kobayashi, S. et al. (2000) J. Biol. Chem. 275, 10723-10726) were maintained in RPMI1640 medium supplemented with 10% fetal bovine serum (Bio Whittaker). The cells were seeded at 5 x 10⁴ cells/well in a 96-well plate, and incubated for 24 h, then the medium was replaced with fresh medium containing 1,000 nM Reg protein, 25 ng/ml hepatocyte growth factor (HGF; Sigma), or both of them. After a further 24-h incubation, the cells were harvested, WST-1 cleavage by mitochondrial dehydrogenases in viable cells were measured using a Cell Proliferation Reagent WST-1 (Roche ) as described in Kobayashi, S. et al. (2000) J. Biol. Chem., 275, 10723-10726 and Akiyama, T. et al. (2001) Proc. Natl. Acad. Sci. USA 98, 48-53.

As shown in Fig. 5, the combined addition of HGF and Reg protein significantly increased the number of RINm5F cells, whereas HGF added alone did not. The addition of 1,000 nM Reg protein to the culture media of RINm5F cells, #1, #6, and #24, which express Reg protein receptor, induced not only proliferation but also apoptosis of the cells (Kobayashi, S. et al. (2000) J. Biol. Chem. 275, 10723-10726). Furthermore, the addition of HGF alone was ineffective in increasing the cell number, suggesting that HGF suppressed apoptosis caused by 1,000 nM Reg protein, and therefore the combination use of Reg protein and HGF increased the cell number.

### [Example 5] Apoptosis-suppressive effect of HGF

Rat insulinoma RINm5F cell lines, #1, #6, and #24, which stably express Reg protein receptor (Kobayashi, S. et al. (2000) J. Biol. Chem. 275, 10723-10726) were similarly cultured as in Example 4 and 1,000 nM Reg protein and HGF at varied concentrations, 0, 2.5, and 25 ng/ml were added thereto. Twenty-four hours after the addition of Reg protein/HGF, apoptosis was measured by TUNEL method using an Apoptosis Screening kit (Wako Pure Chemical Industries Ltd., Osaka, Japan) as described in Kobayashi, S. et al. (2000) J. Biol. Chem. 275, 10723-10726.

As shown in Fig. 6, in every cell line, HGF concentration-dependently inhibited apoptosis induced by 1,000 nM Reg protein, indicating that HGF inhibited apoptosis induced by 1,000 nM Reg protein and therefore Reg protein/HGF increased the cell number as observed in Example 1.

### [Example 6] Apoptosis-suppressive effect of IL-6, glucocorticoid, and poly(ADP-ribose) synthetase inhibitor

Rat insulinoma RINm5F cell lines, #1, #6, and #24, which stably express the Reg protein receptor (Kobayashi, S. et al. (2000) J. Biol. Chem. 275, 10723-10726) were similarly cultured as in Example 1 and 1,000 nM Reg protein, 20 ng/ml IL-6 (Genzyme; 5,000 U/ml) as a cytokine whose receptor is gp130, 100 nM dexamethasone as a glucocorticoid , and nicotinamide (Wako Pure Chemical Industries Ltd. , Osaka, Japan: 10 mM) as a poly (ADP-ribose) synthetase (PARP) inhibitor, were added. Twenty-four hours after the addition, apoptosis was measured by TUNEL method using an Apoptosis Screening kit (Wako Pure Chemical Industries Ltd., Osaka, Japan) as described in Kobayashi, S. et al. (2000) J. Biol. Chem. 275, 10723-10726.

A as shown in Fig. 7, the combined addition of IL-6, dexamethasone, and the PARP inhibitor such as nicotinamide suppressed apoptosis induced by Reg protein. PARP inhibitors such as nicotinamide reportedly inhibit acute apoptosis of insulin producing cells (Yamamoto, H. et al. (1981) Nature 294, 284-286; Uchigata, Y. et al. (1982) J. Biol. Chem. 257, 6084-6088; Okamoto, H. (1985) BioEssays 2, 15-21). In this experiment, however, the PARP inhibitor was did not inhibit apoptosis induced by Reg protein. The result suggest that apoptosis caused by Reg protein and cell death caused by streptozotocin, alloxan, or such are induced by different mechanisms, and also that IL-6 and glucocorticoid, which induce Reg protein expression, inhibit apoptosis caused by high concentrations of Reg protein by some mechanism, thereby assisting the cell proliferation by Reg protein.

### [Example 7] Induction of HGF expression by IL-6, glucocorticoid, and PARP inhibitor

Rat insulinoma RINm5F cells were seeded at 1.5 x 10⁵ cells/ml in a 25-cm² culture bottle. After 24-h incubation, the medium was replaced with fresh medium containing 100 nM dexamethasone, 20 ng/ml IL-6 (5,000 U/ml), 10 mM nicotinamide, or combinations thereof. After a further 24-h incubation, total RNA was prepared using cesium trifluoroacetate as described in Takasawa, S. et al. (1993) J. Biol. Chem. 268, 26052-26054 and Akiyama, T. et al. (2001) Proc. Natl. Acad. Sci. USA 98, 48-53. RT-PCR was performed using this RNA as a template and primers corresponding to nucleotides 1,556-1,575 and 2,208-2,227 of rat HGF mRNA (Okajima, A. et al. (1990) Eur. J. Biochem. 193, 375-381) as described in Sun, W. et al. (1999) Brain Res. 851, 46-53, to detect the expression of HGF gene. PCR was carried out by a two-step protocol as described in Sun, W. et al. (1999) Brain Res. 851, 46-53, with 33 cycles of the first step [denaturation (95°C, 40 s), primer annealing (60°C, 40 s), and primer elongation (72°C, 1 min)] followed by the 5 cycles of the second step [denaturation (85°C, 40 s), primer annealing (60°C, 40 s), and primer elongation (72°C, 1 min)].

As clearly shown in Fig. 8, the combined addition of IL-6 and dexamethasone induced the expression of HGF gene that was usually not expressed, and, moreover, the addition of the PARP inhibitor such as nicotinamide to IL-6/dexamethasone further enhanced HGF gene expression. These results suggested that the apoptosis inhibition by IL-6/dexamethasone observed in Example 6 was mediated by induction of HGF gene expression.

### [Example 8] Induction of HGF transcription by IL-6, glucocorticoid and PARP inhibitor

The 5' upstream region (-1336 to +85) of rat HGF gene was inserted into the multicloning site of pGL3-Basic (Promega) to prepare a luciferase reporter gene construct. Rat insulinoma RINm5F cells were seeded at 1 x 10⁵ cells/well in a 24-well plate, incubated for 2 days, then, transfected with the test luciferase reporter construct using DMRIE-C^{™} (Life Technologies, Inc.) as described in Akiyama, T. et al. (2001) Proc. Natl. Acad. Sci. USA 98, 48-53. Twenty-four hours after transfection, 20 ng/ml IL-6, 100 nM dexamethasone (Dx), and 10 mM nicotinamide (NA) were added to the culture medium, and further incubated for 24 h. Then, the cells were harvested in 1 ml of ice-cold phosphate-buffered sa Line (PBS), washed twice with PBS, and cell extracts were prepared in extraction buffer (0.1 M potassium phosphate, pH 6.8, 1 mM DTT). The protein concentration was determined using a Coomassie reagent kit (pierce) and bovine serum albumin as a standard. Luciferase activity was determined using a chemiluminescence substrate and a Pica Gene^{™} Luminescence Kit (Toyo Ink Mfg. Co, Ltd., Tokyo, Japan). β-Galactosidase activity was measured using an Aurora^{™} GAL-XE (ICN).

As shown in Fig. 9, the combined addition of IL-6 and dexamethasone induced the transcriptional activity of HGF gene. Moreover, the combined addition of a poly(ADP-ribose) synthetase inhibitor such as nicotinamide to IL-6/dexamethasone further enhanced this transcriptional induction, indicating that HGF mRNA induction shown in Fig. 8 occurred at the transcriptional level.

### [Example 9] Identification of gene sequence essential for induction of HGF gene expression by IL-6 and glucocorticoid (1)

Various lengths of 5' upstream regions HGF gene (Okajima, A., Miyazawa, K. and Kitamura, N. (1993) Eur. J. Biochem. 213, 113-119 (GenBank Ac. No. D14341)) were inserted into the multicloning site of pGL3-Basic (Promega) to prepare lucif erase reporter gene constructs (Fig. 10). Rat insulinoma RINm5F cells were seeded at 1 x 10⁵ cells/well in a 24-well plate, and incubated for 2 days. Then, test luciferase reporter constructs were introduced into the cells using DMRIE-C^{™} (Life Technologies, Inc.) as described in Akiyama, T. et al. (2001) Proc. Natl. Acad. Sci. USA 98, 48-53, and, 24 h after the introduction, 20 ng/ml IL-6 and 100 nM dexamethasone (Dx) were added to the culture medium, and further incubated for 24 h. Then, the cells were harvested in 1 ml of ice-cold phosphate-buffered saline (PBS), washed twice with PBS, and extracts were prepared in extraction buffer (0.1 M potassium phosphate, pH 6.8, 1 mM DTT). The protein concentration was determined using a Coomassie reagent kit (Pierce) and bovine serum albumin as a standard. Luciferase activity was determined using a chemiluminescence substrate and a Pica Gene^{™} Luminescence Kit (Toyo Ink Mfg. Co. Ltd., Tokyo, Japan). β-Galactosidase activity was measured using an Aurora^{™} GAL-XE (ICN).

As shown in Fig. 10, the combined addition of IL-6 and dexamethasone induced the transcriptional activity in the plasmids containing the upstream regions up to -96 nucleotide from the transcription initiation point of HGF gene, by similarly as the plasmid containing the upstream region up to -1,336 nucleotide. However, IL-6 and dexamethasone were ineffective in inducing the transcriptional activity in the plasmids containing the upstream region up to -92 or 87 nucleotide. Analysis using TRANSFAC computer program (Heinemeyer, T. et al. (1998) Nucleic Acids Res. 26, 362-367) found only the binding sequence (TTMCCRKAA) for the STAT transcription factor family in the 96 to 88 nucleotide upstream region of HGF gene as a known transcriptional factor binding sequence.

### [Example 10] Identification of gene sequence essential for induction of HGF gene expression (2)

To investigate a possibility that the gene sequence essential for the induction of HGF gene expression shown in Fig. 10 is the binding sequence (TTMCCRKAA) for the STAT transcription factor family, a variant of the reporter gene construct, which had been prepared by inserting the 5' upstream region (-1336 to +85) of rat HGF gene into the multicloning site of pGL3-Basic, was prepared by replacing the first and second nucleotides (TT) of the binding sequence, which were thought essential for binding of the STAT transcription factor family, with CC. Rat insulinoma RINm5F cells were seeded at 1 x 10⁵ cells/well in a 24-well plate, and incubated for 2 days. Then, test luciferase reporter gene constructs (wild type and variant reporter gene constructs) were introduced into the cells using DMRIE-C^{™} (Life Technologies, Inc.) as described in Akiyama, T. et al. (2001) Proc. Natl. Acad. Sci. USA 98, 48-53. Twenty-four h after the introduction, 20 ng/ml IL-6 and 100 nM dexamethasone (Dx) were added to the culture medium, and further incubated for 24 h. Then, the cells were harvested in 1 ml of ice-cold phosphate-buffered saline (PBS), washed twice with PBS, and extracts were prepared in extraction buffer (0.1 M potassium phosphate, pH 6.8, 1 mM DTT). The protein concentration was determined using a Coomassie reagent kit (Pierce) and bovine serum albumin as a standard. Luciferase activity was determined using a chemiluminescence substrate and a Pica Gene^{™} Luminescence Kit (Toyo Ink Mfg. Co, Ltd, Tokyo, Japan). β-Galactosidase activity was measured using an Aurora^{™} GAL-XE (TCN).

As shown in Fig. 11, the combined addition of IL-6 and dexamethasone induced the transcriptional activity in the wild type reporter gene construct, while the variant reporter gene construct with only two- nucleotide change did not, indicating that the -96 to -88 sequence TTACCGTAA matching to the binding sequence for STAT transcription factor family is essential for the transcriptional induction of HGF gene by IL-6 and dexamethasone, and also that any member of the STAT transcription factor family may bind to this region.

### Industrial Applicability

The present invention provides Reg gene and/or HGF gene expression inducers comprising cytokine whose receptor is gp130 and glucocorticoid. The use of the Reg gene expression inducer of this invention enables the remarkable induction of Reg gene expression in β-cells, thereby promotes β-cell proliferation. Furthermore, the induction of HGF gene expression enables the suppression of apoptosis, especially one occurrable during β-cell proliferation promoted by Reg, thereby enhancing cell proliferation effects. Reg gene and/or HGF gene expression inducers of this invention can be used to treat and prevent diseases such as diabetes. The addition of a PARP inhibitor as the active ingredient to Reg gene or HGF gene expression inducers of this invention can further enhance the induction effects. Moreover, the present invention provides a method of screening for candidate compounds for Reg gene expression inducers. This method is **characterized in that** effects of test samples on the binding of PARP to the 5' upstream region of Reg gene are detected using a reporter gene. This invention also provides a method of screening for candidate compounds for HGF gene expression inducers . This method is **characterized in that** effects of test samples are detected by a reporter gene assay using the IL-6/Dx responsive sequence. This invention enables an efficient screening for drugs that induce the expression of these genes using a reporter activity as an indicator.

## Claims

1. Use of a cytokine whose receptor is gp 130 in combination with a glucocorticoid in preparation of a medicament for proliferating pancreatic 8-cells of Langerhans' islets.

2. Use of a cytokine whose receptor is gp130 in combination with a glucocorticoid in preparation of a medicament for suppressing apoptosis of pancreatic β-cells of Langerhans' islets.

3. Use of hepatocyte growth factor (HGF) or a nucleic acid coding for HGF in combination with Reg protein or a nucleic acid coding for Reg protein in preparation of a medicament for proliferating pancreatic β-cells of Langerhans' islets.

4. Use of hepatocyte growth factor (HGF) or a nucleic acid coding for HGF in combination with Reg protein or a nucleic acid coding for Reg protein in preparation of a medicament for suppressing apoptosis of pancreatic β-cells of Langerhans' islets.

5. Use of claims 1 or 2, wherein said cytokine is any one of IL-6, IL-11, LIF, CNTF, OSM or CT-1.

6. Use of claims 1 or 2, wherein said cytokine is IL-6.

7. Use of claims 1 or 2, wherein said glucocorticoid is dexamethasone.

8. Use of claims 1 or 2, further comprising use of a poly(ADP-ribose) synthetase/polymerase (PARP) inhibitor.

9. Use of claim 8, wherein said poly(ADP-ribose) synthetase/polymerase (PARP) inhibitor is nicotinamide or 3-aminobenzamide.

10. Use of any one of claims 1 to 9, wherein said medicament is used in the treatment or prophylaxis of diabetes.

11. In vitro method of proliferating pancreatic β-cells of Langerhans' islets, said method comprises administrating a cytokine whose receptor is gp130 in combination with a glucocorticoid.

12. In vitro method of suppressing apoptosis of pancreatic β-cells of Langerhans' islets, said method comprises administrating a cytokine whose receptor is gp130 in combination with a glucocorticoid.

13. In vitro method of proliferating pancreatic β-cells of Langerhans' islets, said method comprises administrating hepatocyte growth factor (HGF) or a nucleic acid coding for HGF in combination with Reg protein or a nucleic acid coding for Reg protein.

14. In vitro method of suppressing apoptosis of pancreatic β-cells of Langerhans' islets, said method comprises administrating hepatocyte growth factor (HGF) or a nucleic acid coding for HGF in combination with Reg protein or a nucleic acid coding for Reg protein.

15. The method of claims 11 or 12, wherein said cytokine is any one of IL-6, IL-11, LIF, CNTF, OSM or CT-1.

16. The method of claims 11 or 12, wherein said cytokine is IL-6.

17. The method of claims 11 or 12, wherein said glucocorticoid is dexamethasone.

18. The method of claims 11 or 12, further comprising administrating a poly(ADP-ribose) synthetase/polymerase (PARP) inhibitor.

19. The method of claim 18, wherein said poly(ADP-ribose) synthetase/polymerase (PARP) inhibitor is nicotinamide or 3-aminobenzamide.

## Patentansprüche

1. Verwendung eines Cytokins, dessen Rezeptor gp130 ist, in Kombination mit einem Glucocorticoid bei der Herstellung eines Medikaments zur Vermehrung von pankreatischen β-Zellen der Langerhansschen Inseln.

2. Verwendung eines Cytokins, dessen Rezeptor gp130 ist, in Kombination mit einem Glucocorticoid bei der Herstellung eines Medikaments zur Unterdrückung der Apoptose von pankreatischen β-Zellen der Langerhansschen Inseln.

3. Verwendung des Hepatocytenwachstumsfaktors (HGF) oder einer für HGF codierenden Nucleinsäure in Kombination mit Reg-Protein oder einer für Reg-Protein codierenden Nucleinsäure bei der Herstellung eines Medikaments zur Vermehrung von pankreatischen β-Zellen der Langerhansschen Inseln.

4. Verwendung des Hepatocytenwachstumsfaktors (HGF) oder einer für HGF codierenden Nucleinsäure in Kombination mit Reg-Protein oder einer für Reg-Protein codierenden Nucleinsäure bei der Herstellung eines Medikaments zur Unterdrückung der Apoptose von pankreatischen β-Zellen der Langerhansschen Inseln.

5. Verwendung gemäß Anspruch 1 oder 2, wobei irgend eines von Cytokin IL-6, IL-11, LIF, CNTF, OSM oder CT-1 ist.

6. Verwendung gemäß Anspruch 1 oder 2, wobei es sich bei dem Cytokin um IL-6 handelt.

7. Verwendung gemäß Anspruch 1 oder 2, wobei es sich bei dem Glucocorticoid um Dexamethason handelt.

8. Verwendung gemäß Anspruch 1 oder 2, die weiterhin die Verwendung eines Poly(ADP-Ribose)-Synthetase/Polymerase(PARP)-Inhibitors umfasst.

9. Verwendung gemäß Anspruch 8, wobei es sich bei dem Poly(ADP-Ribose)-Synthetase/Polymerase(PARP)-Inhibitor um Nicotinamid oder 3-Aminobenzamid handelt.

10. Verwendung gemäß einem der Ansprüche 1 bis 9, wobei das Medikament bei der Behandlung oder Prophylaxe von Diabetes verwendet wird.

11. In-vitro-Verfahren zur Vermehrung von pankreatischen β-Zellen der Langerhansschen Inseln, wobei das Verfahren das Verabreichen eines Cytokins, dessen Rezeptor gp130 ist, in Kombination mit einem Glucocorticoid umfasst.

12. In-vitro-Verfahren zur Unterdrückung der Apoptose von pankreatischen β-Zellen der Langerhansschen Inseln, wobei das Verfahren das Verabreichen eines Cytokins, dessen Rezeptor gp130 ist, in Kombination mit einem Glucocorticoid umfasst.

13. In-vitro-Verfahren zur Vermehrung von pankreatischen β-Zellen der Langerhansschen Inseln, wobei das Verfahren das Verabreichen des Hepatocytenwachstumsfaktors (HGF) oder einer für HGF codierenden Nucleinsäure in Kombination mit Reg-Protein oder einer für Reg-Protein codierenden Nucleinsäure umfasst.

14. In-vitro-Verfahren zur Unterdrückung der Apoptose von pankreatischen β-Zellen der Langerhansschen Inseln, wobei das Verfahren das Verabreichen des Hepatocytenwachstumsfaktors (HGF) oder einer für HGF codierenden Nucleinsäure in Kombination mit Reg-Protein oder einer für Reg-Protein codierenden Nucleinsäure umfasst.

15. Verfahren gemäß Anspruch 11 oder 12, wobei irgend eines von Cytokin IL-6, IL-11, LIF, CNTF, OSM oder CT-1 ist.

16. Verfahren gemäß Anspruch 11 oder 12, wobei es sich bei dem Cytokin um IL-6 handelt.

17. Verfahren gemäß Anspruch 11 oder 12, wobei es sich bei dem Glucocorticoid um Dexamethason handelt.

18. Verfahren gemäß Anspruch 11 oder 12, das weiterhin das Verabreichen eines Poly(ADP-Ribose)-Synthetase/Polymerase(PARP)-Inhibitors umfasst.

19. Verfahren gemäß Anspruch 18, wobei es sich bei dem Poly(ADP-Ribose)-Synthetase/Polymerase(PARP)-Inhibitor um Nicotinamid oder 3-Aminobenzamid handelt.

## Revendications

1. Utilisation d'une cytokine dont le récepteur est gp-130, en combinaison avec un glucocorticoïde, dans la préparation d'un médicament conçu pour faire proliférer les cellules bêta des ilots de Langerhans du pancréas.

2. Utilisation d'une cytokine dont le récepteur est gp-130, en combinaison avec un glucocorticoïde, dans la préparation d'un médicament conçu pour empêcher l'apoptose des cellules bêta des ilots de Langerhans du pancréas.

3. Utilisation du facteur de croissance des hépatocytes (HGF) ou d'un acide nucléique codant le facteur HGF, en combinaison avec la protéine Reg ou un acide nucléique codant la protéine Reg, dans la préparation d'un médicament conçu pour faire proliférer les cellules bêta des ilots de Langerhans du pancréas.

4. Utilisation du facteur de croissance des hépatocytes (HGF) ou d'un acide nucléique codant le facteur HGF, en combinaison avec la protéine Reg ou un acide nucléique codant la protéine Reg, dans la préparation d'un médicament conçu pour empêcher l'apoptose des cellules bêta des ilots de Langerhans du pancréas.

5. Utilisation conforme à la revendication 1 ou 2, dans laquelle ladite cytokine est l'une des suivantes : IL-6, IL-11, LIF, CNTF, OSM et CT-1.

6. Utilisation conforme à la revendication 1 ou 2, dans laquelle ladite cytokine est de l'IL-6.

7. Utilisation conforme à la revendication 1 ou 2, dans laquelle ledit glucocorticoïde est de la dexaméthasone.

8. Utilisation conforme à la revendication 1 ou 2, qui comporte en outre l'utilisation d'un inhibiteur de poly(ADP-ribose) synthétase/polymérase (PARP).

9. Utilisation conforme à la revendication 8, dans laquelle ledit inhibiteur de poly(ADP-ribose) synthétase/polymérase (PARP) est du nicotinamide ou du 3-amino-benzamide.

10. Utilisation conforme à l'une des revendications 1 à 9, ledit médicament étant utilisé dans le traitement ou la prophylaxie du diabète.

11. Procédé *in vitro* permettant de faire proliférer les cellules bêta des ilots de Langerhans du pancréas, lequel procédé comprend l'administration d'une cytokine dont le récepteur est gp-130, en combinaison avec un glucocorticoïde.

12. Procédé *in vitro* permettant d'empêcher l'apoptose des cellules bêta des ilots de Langerhans du pancréas, lequel procédé comprend l'administration d'une cytokine dont le récepteur est gp-130, en combinaison avec un glucocorticoïde.

13. Procédé *in vitro* permettant de faire proliférer les cellules bêta des ilots de Langerhans du pancréas, lequel procédé comprend l'administration du facteur de croissance des hépatocytes (HGF) ou d'un acide nucléique codant le facteur HGF, en combinaison avec la protéine Reg ou un acide nucléique codant la protéine Reg.

14. Procédé *in vitro* permettant d'empêcher l'apoptose des cellules bêta des ilots de Langerhans du pancréas, lequel procédé comprend l'administration du facteur de croissance des hépatocytes (HGF) ou d'un acide nucléique codant le facteur HGF, en combinaison avec la protéine Reg ou un acide nucléique codant la protéine Reg.

15. Procédé conforme à la revendication 11 ou 12, dans lequel ladite cytokine est l'une des suivantes : IL-6, IL-11, LIF, CNTF, OSM et CT-1.

16. Procédé conforme à la revendication 11 ou 12, dans lequel ladite cytokine est de l'IL-6.

17. Procédé conforme à la revendication 11 ou 12, dans lequel ledit glucocorticoïde est de la dexaméthasone.

18. Procédé conforme à la revendication 11 ou 12, qui comporte en outre l'utilisation d'un inhibiteur de poly(ADP-ribose) synthétase/polymérase (PARP).

19. Procédé conforme à la revendication 18, dans lequel ledit inhibiteur de poly(ADP-ribose) synthétase/polymérase (PARP) est du nicotinamide ou du 3-amino-benzamide.


### SEQUENCE LISTING

<110> Okamoto, Hiroshi
<120> Agents for proliferating pancreatic beta-cells of the islets of langerhans, agents for suppressing apoptosis of said cells, and screening of candidate compounds for these agents
<130> P62605EP00
<140> 01934500.8
   <141> 2002-12-30
<150> JP 2000-170447
   <151> 2000-06-02
<150> JP 2001-54072
   <151> 2001-02-28
<160> 25
<170> PatentIn Ver. 2.1
<210> 1
   <211> 31
   <212> DNA
   <213> Rattus norvegicus
<220>
   <221> misc_feature
   <222> (1)..(31)
   <223> /note="IL-6/dexamethasone-sensitive Reg promoter"
<400> 1
   gatattcctt gcccctccca tttttcactc a 31
<210> 2
   <211> 31
   <212> DNA
   <213> Mus musculus
<220>
   <221> misc_feature
   <222> (1)..(31)
   <223> /note="IL-6/dexamethasone-sensitive Reg promoter"
<400> 2
   gacatttctt gctcctccca tttttcaccc a 31
<210> 3
   <211> 31
   <212> DNA
   <213> Homo sapiens
<220>
   <221> misc_feature
   <222> (1)..(31)
   <223> /note="IL-6/dexamethasone-sensitive Reg promoter"
<400> 3
   ctcaccgctt gcccccaccc cttttgctta a 31
<210> 4
   <211> 33
   <212> DNA
   <213> Homo sapiens
<220>
   <221> misc_feature
   <222> (1)..(33)
   <223> /note="IL-6/dexamethasone-sensitive Reg promoter"
<400> 4
   gatccttact ccttgctcca cccattgttt ata 33
<210> 5
   <211> 22
   <212> DNA
   <213> Artificial sequence
<220>
   <223> Description of Artificial sequence:
   oligonucleotide, probe 1
<220>
   <221> misc_feature
   <222> (1)..(22)
<400> 5
   ttccttgccc ctcccatttt tc 22
<210> 6
   <211> 22
   <212> DNA
   <213> Artificial Sequence
<220>
   <223> Description of Artificial Sequence:
   oligonucleotide, probe M1
<220>
   <221> misc_feature
   <222> (1)..(22)
<400> 6
   ttccttgccc ctaacatttt tc 22
<210> 7
   <211> 22
   <212> DNA
   <213> Artificial Sequence
<220>
   <223> Description of Artificial Sequence:
   oligonucleotide, probe M2
<220>
   <221> misc_feature
   <222> (1)..(22)
<400> 7
   ttccttgccc cgcccatttt tc 22
<210> 8
   <211> 22
   <212> DNA
   <213> Artificial Sequence
<220>
   <223> Description of Artificial Sequence:
   oligonucleotide, probe M3
<220>
   <221> misc_feature
   <222> (1)..(22)
<400> 8
   ttccttgccc cacccatttt tc 22
<210> 9
   <211> 22
   <212> DNA
   <213> Artificial Sequence
<220>
   <223> Description of Artificial sequence:
   oligonucleotide, Sp1
<220>
   <221> misc_feature
   <222> (1)..(22)
<400> 9
   attcgatcgg ggcggggcga gc 22
<210> 10
   <211> 25
   <212> DNA
   <213> Artificial sequence
<220>
   <223> Description of Artificial Sequence:
   oligonucleotide, GKLF
<220>
   <221> misc_feature
   <222> (1)..(25)
<400> 10
   aggagaaaga agggcgtagt atcta 25
<210> 11
   <211> 28
   <212> DNA
   <213> Artificial Sequence
<220>
   <223> Description of Artificial Sequence:
   oligonucleotide, IK-BS4
<220>
   <221> misc_feature
   <222> (1)..(28)
<400> 11
   tcagcttttg ggaatgtatt ccctgtca 28
<210> 12
   <211> 21
   <212> DNA
   <213> Rattus norvegicus
<220>
   <221> misc_feature
   <222> (1)..(21)
<400> 12
   tttcttaccg taagagggag t 21
<210> 13
   <211> 225
   <212> DNA
   <213> Rattus norvegicus
<220>
   <221> mist_feature
   <222> (1)..(225)
   <223> /note="Part of HGF gene"
<400> 13
   ggaaggggct ggaagagagt aaagggctgt tgttaaacag tttcttaccg taagagggag 60
   tttagtccta gatctttcca gttaatcaca caacaaactt agctcatcgc aataaaagca 120
   gctcagaacc gaccggcttg caacaggatt ctttcagccc ggcatctcct gcagagggat 180
   cagcctgctc gaactgcaag catgatgtgg gggaccaaac ttctg                 225
<210> 14
   <211> 225
   <212> DNA
   <213> Mus musculus
<220>
   <221> misc_feature
   <222> (1)..(225)
   <223> /note="Part of HGF gene"
<400> 14
   ggaaggggct ggaagagagt aaagggctgt tgttaaacag tttcttaccg taagagggag 60
   ttcagaccta gatctttcca gttaatcaca caacaaactt agctcatcgc aataaaagca 120
   gctcagaacc gaccggtttg caacaggatt ctttcagccc ggcatctcct gcagagggat 180
   cagtctgctc gaactgcaag catgatgtgg gggaccaaac ttctg                 225
<210> 15
   <211> 241
   <212> DNA
   <213> Homo sapiens
<220>
   <221> misc_feature
   <222> (1)..(241)
   <223> /note="Part of HGF gene"
<400> 15
   ggagggggct ggaagagagt aaagggctgt tgttaaacag tttcttaccg taagagggag 60
   ttcagaccta gatctttcca gttaatcaca caacaaactt agctcatcgc aataaaaagc 120
   agctcagagc cgactggctc ttttaggcac tgactccgaa caggattctt tcacccaggc 180
   atctcctcca gagggatccg ccagcccgtc cagcagcacc atgtgggtga ccaaactcct 240
   g                                                                 241
<210> 16
   <211> 12
   <212> DNA
   <213> Rattus norvegicus
<220>
   <221> misc_feature
   <222> (1)..(12)
   <223> /note="PARP binding sequence"
<400> 16
   tgcccctccc at 12
<210> 17
   <211> 12
   <212> DNA
   <213> Homo sapiens
<220>
   <221> misc_feature
   <222> (1)..(12)
   <223> /note="PARP binding sequence"
<400> 17
   gcccccaccc ct 12
<210> 18
   <211> 12
   <212> DNA
   <213> Homo sapiens
<220>
   <221> misc_feature
   <222> (1)..(12)
   <223> /note="PARP binding sequence"
<400> 18
   tgctccaccc at 12
<210> 19
   <211> 9
   <212> DNA
   <213> Artificial Sequence
<220>
   <223> Description of Artificial sequence: STATx
   consensus sequence
<220>
   <221> misc_feature
   <222> (1)..(9)
   <223> /note="M: A or C, R: A or G, K: G or T"
<400> 19
   ttmccrkaa 9
<210> 20
   <211> 9
   <212> DNA
   <213> Artificial Sequence
<220>
   <223> Description of Artificial Sequence: STATx
   consensus sequence
<220>
   <221> misc_feature
   <222> (1)..(9)
<400> 20
   ttaccgtaa 9
<210> 21
   <211> 11
   <212> DNA
   <213> Artificial Sequence
<220>
   <223> Description of Artificial Sequence:
   IL-6/dexamethasone-sensitive part of Reg promoter
<220>
   <221> misc_feature
   <222> (1)..(11)
<400> 21
   tgcccctccc a 11
<210> 22
   <211> 11
   <212> DNA
   <213> Artificial Sequence
<220>
   <223> Description of Artificial Sequence:
   IL-6/dexamethasone-sensitive part of Reg promoter
<220>
   <221> misc_feature
   <222> (1)..(11)
<400> 22
   tgcccctaac a 11
<210> 23
   <211> 11
   <212> DNA
   <213> Artificial Sequence
<220>
   <223> Description of Artificial sequence:
   IL-6/dexamethasone-sensitive part of Reg promoter
<220>
   <221> misc_feature
   <222> (1)..(11)
<400> 23
   tgccccgccc a 11
<210> 24
   <211> 11
   <212> DNA
   <213> Artificial Sequence
<220>
   <223> Description of Artificial sequence:
   IL-6/dexamethasone-sensitive part of Reg promoter
<220>
   <221> misc_feature
   <222> (1)..(11)
<400> 24
   tgccccaccc a 11
<210> 25
   <211> 8
   <212> DNA
   <213> Artificial Sequence
<220>
   <223> Description of Artificial Sequence:
   IL-6/dexamethasone-sensitive part of Reg promoter
<220>
   <221> misc_feature
   <222> (1)..(8)
<400> 25
   cccctccc 8